(19) Europäisches Patentamt
European Patent Office
Office européen des brevets

(11) Publication number : 0 253 468 B1

# (12) EUROPEAN PATENT SPECIFICATION

(45) Date of publication of patent specification : 06.11.91 Bulletin 91/45

(51) Int. Cl.$^5$ : C07D 213/86, C07D 213/87, C07D 241/24, C07D 239/30, A01N 43/40, A01N 43/60, A01N 43/54

(21) Application number : 87303090.2

(22) Date of filing : 09.04.87

## (54) Six-membered heterocyclic derivatives of N'substituted-N,N'-diacylhydrazines.

(30) Priority : 14.07.86 US 885508
19.02.87 US 12380

(43) Date of publication of application : 20.01.88 Bulletin 88/03

(45) Publication of the grant of the patent : 06.11.91 Bulletin 91/45

(84) Designated Contracting States : AT BE CH DE ES FR GB GR IT LI LU NL SE

(56) References cited :
EP-A- 0 030 789
JOURNAL OF THE CHEMICAL SOCIETY, December 1964, pages 4793-4800; D. MACKAY et al.: "An attempt to study reactions of the free benzoyl radical: Reactions of azodibenzoyl and related compounds"

(73) Proprietor : ROHM AND HAAS COMPANY
Independence Mall West
Philadelphia Pennsylvania 19105 (US)

(72) Inventor : Chi-Tung Hsu, Adam
1686 Heebner Way,
Lansdale, Pennsylvania 19446 (US)
Inventor : Phat Le, Dat
125 Washington Avenue,
North Wales, Pennsylvania, 19454 (US)

(74) Representative : Angell, David Whilton et al
ROHM AND HAAS (UK) LTD. European Operations Patent Department Lennig House
2 Mason's Avenue
Croydon CR9 3NB (GB)

EP 0 253 468 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

cinq atomes de carbone nucléaire, dans lesquels les substituants peuvent être un ou deux, les memes ou différents, halo, nitro, alcoyl en $C_1$ à $C_4$, alcoxy en $C_1$ à $C_4$ ou thio-alcoxy en $C_1$ à $C_4$.

4. L'utilisation selon la revendication 3 du composé ou sel dans lequel

X et X' sont O ;

$R^1$ est t-butyl, ,néopentyl (2,2-diméthylpropyl) ou 1,2,2-triméthylpropyl ; et

A et B sont phényl ou phényl substitué dans lesquels les substituants peuvent être un ou deux, les mêmes ou différents, chloro, fluoro, bromo, iodo, nitro, méthyl, éthyl ou trifluorométhyl ; ou pyridyl ou 2,5-pyrazinyl, non substitués ou substitués dans lesquels le substituant peut être halo, nitro, alcoyl en $C_1$ à $C_4$, alcoxy en $C_1$ à $C_4$, ou thio-alcoxy en $C_1$ à $C_4$.

5. L'utilisation selon la revendication 4 du composé ou sel dans lequel

X et X' sont O ;

$R^1$ est t-butyl ; et

A et B sont phényl ou phényl substitué dans lesquels les substituants peuvent être un ou deux, les mêmes ou différents, chloro, fluro, bromo, iodo, nitro, méthyl, éthyl ou trifluorométhyl ; ou pyridyl ou 2,5-pyrazinyl, ou pyridyl substitué par halo, alcoyl en $C_1$ à $C_3$ ou thiométhoxy.

6. L'utilisation selon la revendication 5 du composé ou sel dans lequel

X et X' sont O ;

$R^1$ est t-butyl ; et

A est 2-pyridyl et B est phényl, 3-méthylphényl, 4-fluorophényl ou 2-chloro-4-fluorophényl ; ou

A est 5-bromo-3-pyridyl et B est 4-chlorophényl ; ou

A est 2-chloro-3-pyridyl et B est 3-méthylphényl; ou

A est 2,3-diméthylphenyl et B est 2-chloro-3-pyridyl.

7. L'utilisation selon l'une quelconque des revendications précédentes du composé ou sel en une quantité de 0,0001 à 99 %, de préférence de 0,001 à environ 90 %, en poids de la composition.

8. L'utilisation selon la revendication 7 du composé ou sel en une quantité de 0,01 à 75 % en poids de la composition.

9. L'utilisation selon l'une quelconque des revendications précédentes du composé ou sel pour réaliser une composition insecticide selon la revendication 7 ou 8 (a) contenant additionnellement un agent dispersant, ladite composition étant sous la forme d'une poudre mouillable, ou (b) contenant un vehicule liquide agronomiquement acceptable et un agent dispersant, ladite composition étant sous la forme d'un produit coulant, ou (c) sous la forme d'une poussière, ou (d) contenant en outre un agent liant, ladite composition étant sous la forme d'un granulé ou (e) contenant en outre un agent attractif, ladite composition étant sous la forme d'un appât ou (f) contenant en outre un agent émulsifiant, ladite composition étant sous la forme d'un concentré émulsifiable.

10. L'utilisation d'un composé ou sel tel que défini dans l'une quelconque des revendications 1 à 6, éventuellement dans une composition contenant également un diluant ou véhicule agronomiquement acceptable, pour contrôler les insectes, en mettant en contact lesdits insectes avec une quantité insecticidement efficace dudit composé ou sel.

11. Un procédé mécanique pour améliorer la valeur commerciale et/ou le profit tiré de récoltes vendables de plantes dont la croissance est affectée ou peut vraisemblablement être affectée par des insectes comprenant (1) le chargement dans un récipient, un dispositif de fumigation ou un dispositif de dissémination mécanique d'un composé ou sel insecticide tel que défini dans l'une quelconque des revendications 1 à 6, éventuellement en mélange avec un diluant ou véhicule agronomiquement acceptable, (2) l'utilisation du récipient, fumigateur ou dispositif de dissémination mécanique pour appliquer le composé ou sel insecticide, sous la forme de granules, poussière, fumée, vapeur ou préparation liquide contenant un agent tensio-actif à des plantes en croissance ou à un milieu de croissance dans lequel des plantes croissent ou vont croître, ou aux insectes eux-mêmes, (3) le contrôle de la dose de l'ingrédient actif pendant cette phase d'application pour que le taux d'application du composé insecticide actif soit suffisant pour combattre les insectes, mais soit insuffisant pour produire un effet adverse inacceptable sur les plantes de la récole croissant ou devant croître dans la zone traitée.

12. L'utilisation ou procédé selon la revendication 10 ou 11 qui comprend l'application du composé ou composition à des plantes en croissance ou dans une zone dans laquelle des plantes sont cultivées, en une quantité de 10 g à 10 kg du composé actif par hectare, de préférence de 100 g à 5 kg par hectare.

13. L'utilisation ou procédé selon la revendication 12 dans lequel les insectes sont de l'ordre des lépidoptères.

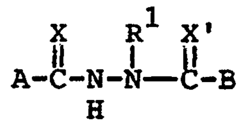

I

wherein

X and X′ are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitroso; nitro; cyano; hydroxy; ($C_1$-$C_6$)alkyl; halo-($C_1$-$C_6$)alkyl; cyano-($C_1$-$C_6$)alkyl; ($C_1$-$C_6$)alkoxy; halo-($C_1$-$C_6$)alkoxy; ($C_1$-$C_6$)alkoxy-($C_1$-$C_6$)alkyl having independently the stated number of carbon atoms in each alkyl group; ($C_1$-$C_6$)alkoxy-($C_1$-$C_6$)alkoxy having independently the stated number of carbon atoms in each alkyl group; $-OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; carboxy; $-ZCO_2R'$ group; -COR group; halo-($C_1$-$C_6$)alkyl-carbonyl; cyano-($C_1$-$C_6$)alkyl-carbonyl; nitro-($C_1$-$C_6$)alkyl-carbonyl; ($C_1$-$C_6$)alkoxy-carbonyl; halo-($C_1$-$C_6$)alkoxy-carbonyl; -OCOR group; $NH_2$; NHZ; NZZ′; amino substituted with hydroxy, ($C_1$-$C_4$)alkoxy or ($C_1$-$C_4$)alkylthio groups; -CONRR′ group; -OCONRR′ group; -NRCOR′ group; $-NRCO_2R'$ group; -OCONRCOR′ group; sulfhydryl; ($C_1$-$C_6$)alkylthio; halo-($C_1$-$C_6$)alkylthio; -NRCSR′ group; -SCOR group; unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)- alkoxy-carbonyl, ($C_1$-$C_4$)alkanoyloxy, $NH_2$, NHZ or NZZ′: phenoxy where the phenyl ring is unsubstituted or substituted with one to three of the same or different halo, cyano, nitro, hydroxy, ($C_1$-$C_4$)alkyl, ($C_1$-$C_4$)- alkoxy, carboxy, ($C_1$-$C_4$)alkoxy-carbonyl, ($C_1$-$C_4$)alkanoyloxy, $NH_2$, NHZ or NZZ′; benzoyl where the phenyl ring is unsubstituted or substituted with one to three of the same or different halo, cyano, nitro, hydroxy, ($C_1$-$C_4$)alkyl, ($C_1$-$C_4$)- alkoxy, carboxy, ($C_1$-$C_4$)alkoxy-carbonyl, ($C_1$-$C_4$)alkanoyloxy, $NH_2$, NHZ or NZZ′; phenoxycarbonyl where the phenyl ring is unsubstituted or substituted with one to three of the same or different halo, cyano, nitro, hydroxy, ($C_1$-$C_4$)alkyl, ($C_1$-$C_4$)- alkoxy, carboxy, ($C_1$-$C_4$)alkoxy-carbonyl, ($C_1$-$C_4$)alkanoyloxy, $NH_2$, NHZ or NZZ′; phenylthio where the phenyl ring is unsubstituted or substituted with one to three of the same or different halo, cyano, nitro, hydroxy, ($C_1$-$C_4$)alkyl, ($C_1$-$C_4$)- alkoxy, carboxy, ($C_1$-$C_4$)alkoxy-carbonyl, ($C_1$-$C_4$)alkanoyloxy, $NH_2$, NHZ or NZZ′; or when two adjacent positions on the phenyl ring are substituted with alkoxy groups, these groups may be joined to form together with the carbon atoms to which they are attached, a 5 or 6 membered dioxolano or dioxano heterocyclic ring; or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; carboxy; ($C_1$-$C_6$)alkoxy-carbonyl; $-ZCO_2R'$ group; -CONRR′ group; $NH_2$; NHZ; NZZ′; -NRCOR′ group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl, $NH_2$, NHZ or NZZ′;

where R and R′ are hydrogen or ($C_1$-$C_6$)alkyl; Z and Z′ are ($C_1$-$C_6$)alkyl; "amino" means NRR′; or an agronomically acceptable salt thereof; where one of A or B is an unsubstituted or substituted six-membered heterocycle as defined above; provided that B is not 4-alkyl substituted phenyl or 3-alkoxy substituted phenyl when A is 2-pyridyl.

The invention also comprises insecticidal compositions containing these compounds, for example in an amount of 0.0001% to 99% by weight, together with agronomically acceptable diluent or carrier, and the use of the compounds and compositions to combat insects.

The term "halo" should be understood as including chloro, fluoro, bromo and iodo. The term "alkyl" by itself or as a part of another substituent, unless otherwise stated, includes straight or branched chain groups such as methyl, ethyl, n-propyl, isopropyl, n-butyl, t-butyl, isobutyl and neopentyl and where indicated higher homologues and isomers such as n-octyl and isooctyl. The term "haloalkyl" by itself or as part of another substituent is an alkyl group of the stated number of carbon atoms having one or more halo atoms bonded thereto such as chloromethyl, 1- or 2-bromoethyl and trifluoromethyl. Analogously, "cyanoalkyl" by itself or as part of another group is an alkyl group of the stated number of carbon atoms having one or more cyano groups bonded thereto; "haloalkoxy" by itself or as part of another group is an alkoxy group of the stated number of carbon atoms having one or more halo atoms bonded thereto such as difluoromethoxy, trifluoromethoxy, 2-fluoroethoxy and 2,2,2-trifluoroethoxy. "Alkenyl" and "alkynyl" by themselves or as part of another substituent comprise

straight and branched chain groups of the stated number of carbon atoms. "Alkadienyl" is a straight or branched chain alkenyl group comprising two carbon-carbon double bonds that can be conjugated such as 1,3-butadienyl, cumulated such as 1,2-propadienyl or isolated such as 1,4-pentadienyl. Representative examples of six-membered heterocycles having one, two, three or four nitrogen atoms and two to five nuclear carbon atoms include 2-pyridyl, 3-pyridyl, 4-pyridyl, 3-pyridazinyl, 4-pyridazinyl, 5-pyridazinyl, 2-pyrimidinyl, 4-pyrimidinyl, 5-pyrimidinyl, 2-pyrazinyl, 3-pyrazinyl, 2-(1,3,5-triazinyl), 3-(1,2,4-triazinyl), 5-(1,2,4-triazinyl), 6-(1,2,4-triazinyl), 4-(1,2,3-triazinyl) and 5-(1,2,3-triazinyl).

Typical compounds within the scope of the present invention include:

N′-t-butyl-N-(benzoyl)-N′-(isonicotinoyl)hydrazine
N′-t-butyl-N-(2-pyridylcarbonyl)-N′-(benzoyl)hydrazine
N′-t-butyl-N-(benzoyl)-N′-(nicotinoyl)hydrazine
N′-t-butyl-N-(nicotinoyl)-N′-(3,4-dichlorobenzoyl)hydrazine
N′-t-butyl-N-(benzoyl)-N′-(2-pyridylcarbonyl)hydrazine
N′-t-butyl-N-(nicotinoyl)-N′-(benzoyl)hydrazine
N′-t-butyl-N-(2-pyridylcarbonyl)-N′-(2-nitrobenzoyl)hydrazine
N′-t-butyl-N-(2-pyridylcarbonyl)-N′-(2-bromobenzoyl)hydrazine
N′-t-butyl-N-(4-ethylbenzoyl)-N′-(2-pyridylcarbonyl)hydrazine
N′-t-butyl-N-(4-trifluoromethoxybenzoyl)-N′-(2-pyridylcarbonyl)hydrazine
N′-t-butyl-N-(2-pyridylcarbonyl)-N′-(4-methylbenzoyl)hydrazine
N′-t-butyl-N-(2-pyridylcarbonyl)-N′-(3,4-dichlorobenzoyl)hydrazine
N′-t-butyl-N-(benzoyl)-N′-(pyrazinylcarbonyl)hydrazine
N′-t-butyl-N-(2-pyridylcarbonyl)-N′-(3,5-dimethylbenzoyl)hydrazine
N′-t-butyl-N-(5-bromonicotinoyl)-N′-(4-chlorobenzoyl)hydrazine
N′-t-butyl-N-(pyrazinylcarbonyl)-N′-(benzoyl)hydrazine
N′-t-butyl-N-(isonicotinoyl)-N′-(benzoyl)hydrazine
N′-t-butyl-N-(2-pyridylcarbonyl)-N′-(2-iodobenzoyl)hydrazine
N′-t-butyl-N-(2-pyridylcarbonyl)-N′-(2,4-dichlorobenzoyl)hydrazine
N′-t-butyl-N-(2-pyridylcarbonyl)-N′-(4-fluorobenzoyl)hydrazine
N′-t-butyl-N-(2-pyridylcarbonyl)-N′-(2-trifluoromethylbenzoyl)hydrazine
N′-t-butyl-N-(2-pyridylcarbonyl)-N′-(3-nitrobenzoyl)hydrazine
N′-t-butyl-N-(isonicotinoyl)-N′-(3-methylbenzoyl)hydrazine
N′-t-butyl-N-(2-bromonicotinoyl)-N′-(4-chlorobenzoyl)hydrazine
N′-t-butyl-N-(2-methylisonicotinoyl)-N′-(2-chlorobenzoyl)hydrazine
N′-t-butyl-N-(4-nitro-2-pyridylcarbonyl)-N′-(benzoyl)hydrazine
N′-t-butyl-N-(2,5-dichloronicotinoyl)-N′-(3-methylbenzoyl)hydrazine
N′-t-butyl-N-(5-methylnicotinoyl)-N′-(4-nitrobenzoyl)hydrazine
N′-t-butyl-N-(2-pyrimidinylcarbonyl)-N′-(3-methylbenzoyl)hydrazine
N′-t-butyl-N-(4-pyrimidinylcarbonyl)-N′-(2-bromobenzoyl)hydrazine
N′-t-butyl-N-(2-pyrimidinylcarbonyl)-N′-(4-chlorobenzoyl)hydrazine
N′-t-butyl-N-(2,3-dimethylbenzoyl)-N′-(nicotinoyl)hydrazine
N′-t-butyl-N-(2,3-dimethylbenzoyl)-N′-(isonicotinoyl)hydrazine
N′-t-butyl-N-(2-methyl-3-chlorobenzoyl)-N′-(2-pyridylcarbonyl)hydrazine
N′-t-butyl-N-(3-pyridazinylcarbonyl)-N′-(benzoyl)hydrazine
N′-t-butyl-N-(4-pyridazinylcarbonyl)-N′-(4-chlorobenzoyl)hydrazine
N′-t-butyl-N-(4-pyridazinylcarbonyl)-N′-(3-methylbenzoyl)hydrazine
N′-t-butyl-N-(1,3,5-triazinyl-2-carbonyl)-N′-(4-chlorobenzoyl)hydrazine
N′-t-butyl-N-(4-ethylbenzoyl)-N′-(2-pyrimidinylcarbonyl)hydrazine
N′-t-butyl-N-(benzoyl)-N′-(2-pyrimidinylcarbonyl)hydrazine
N′-t-butyl-N-(2,3-dimethylbenzoyl)-N′-(4-pyrimidinylcarbonyl)hydrazine
N′-t-butyl-N-(4-methylbenzoyl)-N′-(4-pyridazinylcarbonyl)hydrazine
N′-t-butyl-N-(4-chlorobenzoyl)-N′-(1,3,5-triazinyl-2-carbonyl)hydrazine
N′-t-butyl-N-(3-chlorobenzoyl)-N′-(4-chloro-2-pyrimidinylcarbonyl)hydrazine
N′-isopropyl-N-(benzoyl)-N′-(nicotinoyl)hydrazine
N′-isopropyl-N-(2-chlorobenzoyl)-N′-(2-pyrimidinylcarbonyl)hydrazine
N′-sec-butyl-N-(3-bromobenzoyl)-N′-(isonicotinoyl)hydrazine
N′-t-butyl-N-(2-chloro-3-pyridylcarbonyl)-N′-(3-methylbenzoyl)hydrazine
N′-t-butyl-N-(2,3-dimethylbenzoyl)-N′-(2-chloro-3-pyridylcarbonyl)hydrazine
N′-t-butyl-N-(2-pyridylcarbonyl)-N′-(2-chloro-5-methylbenzoyl)hydrazine

N′-t-butyl-N-(2-chloro-3-pyridylcarbonyl)-N′-(2-chloro-5-methylbenzoyl)hydrazine
N′-t-butyl-N-(2-thiomethoxy-3-pyridylcarbonyl)-N′-(2-methylbenzoyl)hydrazine
N′-t-butyl-N-(2-thiomethoxy-3-pyridylcarbonyl)-N′-(3,4-dichlorobenzoyl)hydrazine
N-t-butyl-N-(2-chloro-3-pyridylcarbonyl)-N′-(benzoyl)hydrazine
N′-t-butyl-N-(2-pyridylcarbonyl)-N′-(2-chloro-4-fluorobenzoyl)hydrazine

Insecticidal compounds of the present invention having very good activity for use in the insecticidal compositions and formulations of the present invention include those where any one or combination of two or more of the substituents conforms to the following definitions:

X and X′ are O or S;

$R^1$ is branched ($C_3$-$C_8$)alkyl;

A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)alkoxy-($C_1$-$C_4$)alkyl; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or

unsubstituted or substituted six-membered heterocycle having one or two nitrogen atoms and 4 to 5 nuclear carbon atoms where the substituents can be one or two of the same or different halo; nitro; ($C_1$-$C_4$)alkyl; ($C_1$-$C_4$)alkoxy; thio-($C_1$-$C_4$)alkoxy; -NDD′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NDD′;

where D and D′ are hydrogen or ($C_1$-$C_4$)alkyl; where one of A or B is an unsubstituted or substituted six-membered heterocycle as defined.

Because of their insecticidal activity, preferred compounds of the present invention for use in the insecticidal compositions and formulations of the present invention include those where any one or combination of two or more of the substituents conforms to the following definitions:

X and X′ are O;

$R^1$ is branched ($C_4$-$C_7$)alkyl;

A and B are phenyl or substituted phenyl where the substituents can be from one to three of the same or different halo, nitro, ($C_1$-$C_4$)alkyl, ($C_1$-$C_4$)alkoxy or halo-($C_1$-$C_4$)alkyl; or

unsubstituted or substituted six-membered heterocycle having one or two nitrogen atoms and four or five nuclear carbon atoms where the substituents can be one or two of the same or different halo, nitro, ($C_1$-$C_4$)alkyl, ($C_1$-$C_4$)alkoxy or thio-($C_1$-$C_4$)alkoxy; where one of A or B is an unsubstituted or substituted six-membered heterocycle as defined above.

Because of their outstanding insecticidal activity, particularly preferred compounds of the present invention for use in the insecticidal compositions and formulations of the present invention include those where any one or combination of two or more of the substituents conforms to the following definitions:

X and X′ are O;

$R^1$ is t-butyl, neopentyl (2,2-dimethylpropyl) or 1,2,2-trimethylpropyl;

A and B are phenyl or substituted phenyl where the substituents can be one or two of the same or different chloro, fluoro, bromo, iodo, nitro, methyl, ethyl or trifluoromethyl; or unsubstituted or substituted pyridyl or 2,5-pyrazinyl where the substituent can be halo, nitro, ($C_1$-$C_4$)alkyl, ($C_1$-$C_4$)alkoxy or thio-($C_1$-$C_4$)alkoxy;

where one of A or B is an unsubstituted or substituted six-membered heterocycle as defined above.

More preferred compounds conform to the following definitions:

X and X′ are O;

$R^1$ is t-butyl; and

A and B are phenyl or substituted phenyl where the substituents can be one or two of the same of different chloro, fluoro, bromo, iodo, nitro, methyl, ethyl or trifluoromethyl; or pyridyl or 2,5-pyrazinyl, or pyridyl substituted with halo, ($C_1$-$C_3$)alkyl or thiomethoxy;

where one of A or B is an unsubstituted or substituted six-membered heterocycle as definied above.

Mos preferred compounds conform to the following definitions.

X and X′ are O;

$R^1$ is t-butyl; and

A is 2-pyridyl and B is phenyl, 3-methylphenyl, 4-fluorophenyl or 2-chloro-4-fluorophenyl; or

A is 5-bromo-3-pyridyl and B is 4-chlorophenyl; or

A is 2-chloro-3-pyridyl and B is 3-methylphenyl; or

A is 2,3-dimethylphenyl and B is 2-chloro-3-pyridyl.

Because of their relatively low activities at low application rates, the less preferred compounds of this invention are those in which:

A is 2-(5-alkyl)-pyridyl or 3-(methylthio)-pyridyl;
and the least preferred are those in which:
A is 2-pyridyl and B is 4-alkyl substituted phenyl, or
A is 2-pyridyl and B is 3-alkoxy substituted phenyl.

Those N'-substituted-N,N'-diacylhydrazines of Formula I which possess acidic or basic functional groups may be further reacted to form novel salts with appropriate bases or acids. These salts also exhibit pesticidal activity. Typical salts are the agronomically acceptable metal salts, ammonium salts and acid addition salts. Among the metal salts are those in which the metal cation is an alkali metal cation such as sodium, potassium or lithium; alkaline earth metal cation such as calcium, magnesium, barium or strontium; or heavy metal cation such as zinc, manganese, cupric, cuprous, ferric, ferrous, titanium or aluminium. The ammonium salts include those in which the ammonium cation has the formular $NR^5R^6R^7R^8$ wherein each of $R^5$, $R^6$, $R^7$ and $R^8$ are independently hydrogen, hydroxy, $(C_1-C_4)$alkoxy, $(C_1-C_{20})$alkyl, $(C_3-C_8)$alkenyl, $(C_3-C_8)$alkynyl, hydroxy-$(C_2-C_8)$alkyl, $(C_2-C_8)$alkoxyalkyl, amino-$(C_2-C_6)$alkyl, halo-$(C_2-C_6)$alkyl, amino, $(C_1-C_4)$alkyl- or di-$(C_1-C_4)$alkylamino, substituted or unsubstutited phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6-membered heterocyclic ring, optionally having up to one additional hereto atom (e.g., oxygen, nitrogen, or sulfur) in the ring, and preferably saturated, such as piperidino, morpholino, pyrrolidino or piperazino, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, $(C_1-C_8)$alkyl, $(C_1-C_4)$alkoxy, hydroxy, nitro, trifluoromethyl, cyano, amino and $(C_1-C_4)$alkylthio. Such substituted phenyl groups preferably have up to two such substituents. Representative ammonium cations include ammonium, dimethylammonium, 2-ethylhexylammonium, bis(2-hydroxy ethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium and 4-methylbenzyltrimethylammonium. Among the acid addition salts are those in which the anion is an agronomically acceptable anion such as hydrochloride, hydrobromide, sulfate, nitrate, perchlorate, acetate and oxalate.

The compounds of this invention or their precursors can be prepared according to the following processes.

Process A

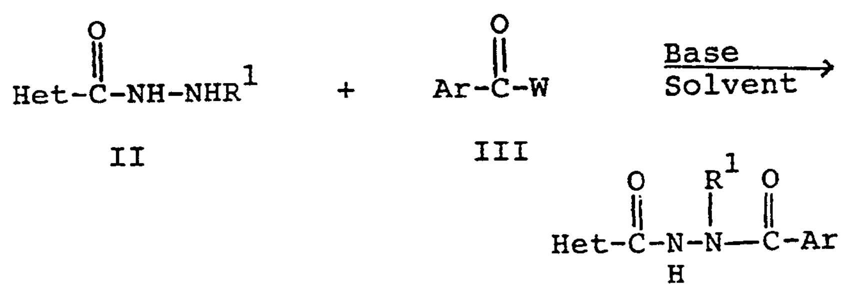

II
III
I

where Het is a six-membered heterocycle as defined above for Formula I, Ar is phenyl as defined above for Formula I, $R^1$ is as defined above for Formula I and W is a good leaving group such as halo, for example, chloro; an alkoxy, for example, ethoxy; a methyl sulfonate ($-OSO_2CH_3$); or an ester, for example, acetate ($-OC(O)CH_3$).

In Process A, a compound of Formula II is reacted with a compound of Formula III in the presence of a base in an inert or substantially inert solvent or mixture of solvents to afford the desired product of Formula I.

Examples of the compounds of Formula III which can be used in the above Process A include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride, methyl benzoate, ethyl benzoate, benzoic acetic anhydride and benzoic methanesulfonic anhydride. The compounds of Formula III are generally commercially available or can be prepared by known procedures.

Suitable solvents for use in the above Process A include water; hydrocarbons such as toluene, xylene, hexane and heptane; alcohols such as methanol, ethanol and isopropanol; glyme, tetrahydrofuran; acetonitrile; pyridine; or haloalkanes such as methylene chloride; or mixture of these solvents.

Preferred solvents are water, toluene, methylene chloride or a mixture of these solvents.

Examples of bases for use in the above Process A include tertiary amines such as triethylamine; pyridine; potassium carbonate; sodium carbonate; sodium bicarbonate; sodium hydroxide; or potassium hydroxide. Preferred bases are sodium hydroxide, or triethylamine.

The compounds of Formula II can be prepared from commercially available compounds by procedures well known to those skilled in the art as described below.

The above Process A can be carried out at temperatures between about -50°C and about 150°C. Preferably, when W is a halo radical, the reaction is carried out between about 0°C and about 30°C. When W is alkoxy, the reaction is preferably carried out between about 100°C and about 150°C. When W is methyl sulfonate, the reaction is preferably carried out between about -20°C to about 20°C. When W is an ester, the reaction is preferably carried out between about 0°C and about 50°C.

Preparation of the compounds of the present invention by Process A is preferably carried out at about atmospheric pressure, although higher or lower pressures can be used if desired.

Substantially equimolar amounts of reactants are preferably used in Process A, although higher or lower amounts can be used if desired.

Generally, about one equivalent of base is used per equivalent of the reactant of Formula III.

Process B

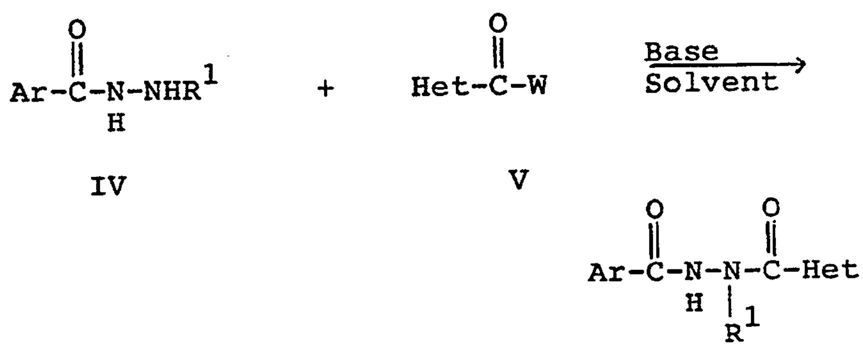


IV

V

I

where Ar is phenyl as defined above for Formula I, $R^1$ is as defined above for Formula I, Het is a six-membered heterocycle as defined above for Formula I and W is a good leaving group such as halo, for example, chloro; an alkoxy, for example, ethoxy; methyl sulfonate ($-OSO_2CH_3$); or an ester, for example, acetate ($-OC(O)CH_3$).

In Process B, an N'-substituted-N-benzoylhydrazine of Formula IV is reacted with a compound of Formula V in the presence of a base in an inert or substantially inert solvent or mixture of solvents to afford the desired product of Formula I.

Examples of the compounds of Formula IV which can be used in the above Process B include N'-isopropyl-N-benzoylhydrazine; N'-sec-butyl-N-benzoylhydrazine; N'-(1-methyl)neopentyl-N-benzoylhydrazine; N'-neopentyl-N-benzoylhydrazine; N'-isobutyl-N-benzoylhydrazine; N'-(1,2,2-trimethylpropyl)-N-benzoylhydrazine; N'-diisopropylmethyl-N-benzoylhydrazine; N'-t-butyl-N-benzoylhydrazine; N'-t-butyl-N-(4-methylbenzoyl)hydrazine; N'-t-butyl-N-(4-chlorobenzoyl)hydrazine;

The compounds of Formula V are generally commercially available or can be prepared from commercially available compounds by procedures well known to those skilled in the art as described below.

Suitable solvents for use in the above Process B include water; hydrocarbons such as toluene, xylene, hexane and heptane; alcohols such as methanol, ethanol and isopropanol; glyme, tetrahydrofuran; acetonitrile; pyridine; or haloalkanes such as methylene chloride; or mixtures of these solvents. Preferred solvents are water, toluene, methylen chloride or a mixture of these solvents.

Examples of bases suitable for use in the above Process B include tertiary amines such as triethylamine: pyridine; potassium carbonate, sodium hydroxide; or potassium hydroxide. Preferred bases are sodium hydroxide, or triethylamine.

The above Process B can be carried out at temperatures between about -50°C and about 150°C. Preferably, when W is a halo radical, the reaction is carried out between about 0°C and about 30°C. When W is alkoxy, the reaction is preferably carried out between about 100°C and about 150°C. When W is methyl sulfonate, the reaction is preferably carried out between about -20°C to about 20°C. When W is an ester, the reaction is preferably carried out between about 0°C and about 50°C.

Preparation of the compounds of the present invention by Process B is preferably carried out at about atmospheric pressure, although higher or lower pressures can be used if desired.

Substantially equimolar amounts of reactants are preferably used in Process B, although higher or lower amounts can be used, if desired.

Generally, about one equivalent of base is used per equivalent of the reactant of Formula V.

<u>Process C</u>

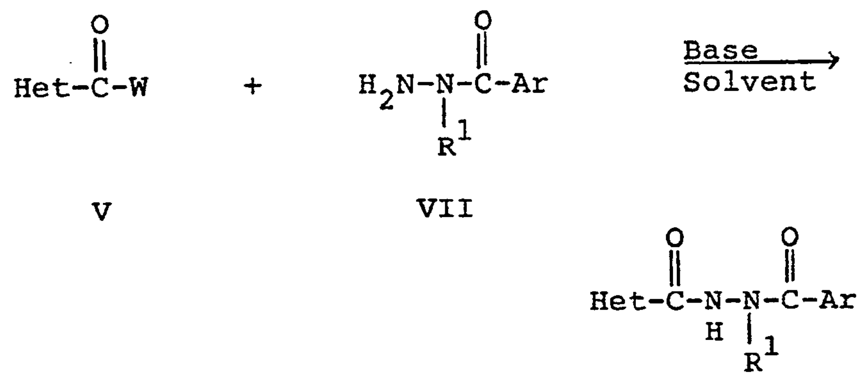


V VII

I

where Ar is phenyl as defined above for Formula I, Het is a six-membered heterocycle as defined above for Formula I, $R^1$ is as defined above for Formula I and W is a good leaving group such as halo, for example, chloro; an alkoxy, for example, ethoxy; methyl sulfonate ($-OSO_2CH_3$); or an ester, for example, acetate ($-OC(O)CH_3$).

In Process C, an N'-substituted-N'-benzoylhydrazine of Formula VII is reacted with a compound of Formula V in the presence of a base in an inert or substantially inert solvent or mixture of solvents to afford the desired product of Formula I.

The compounds of Formula V are generally commercially available or can be prepared from commercially available compounds by procedures well known to those skilled in the art as described below.

Examples of the compounds of Formula VII which can be used in the above Process C include N'-t-butyl-N'-benzoylhydrazine; N'-t-butyl-N'-(3-methylbenzoyl)hydrazine; N'-t-butyl-N'-(4-chlorobenzoyl)hydrazine; N'-t-butyl-N'-(2-fluorobenzoyl)hydrazine; N'-isopropyl-N'-benzoylhydrazine; N'-neopentyl-N'-(4-chlorobenzoyl)hydrazine.

Suitable solvents for use in the above Process C include water; hydrocarbons such as toluene, xylene, hexane and heptane; alcohols such as methanol, ethanol and isopropanol; glyme; tetrahydrofuran, acetonitrile; pyridine; or haloalkanes such as methylene chloride; or mixtures of these solvents. Preferred solvents are water, toluene, methylene chloride or a mixture of these solvents.

Examples of bases suitable for use in the above Process C includes tertiary amines such as triethylamine; pyridine; potassium carbonate; sodium carbonate; sodium bicarbonate; sodium hydroxide; or potassium hydroxide. Preferred bases are sodium hydroxide, or triethylamine.

The above Process C can be carried out at temperatures between about -50°C and about 150°C. Preferably, when W is a halo radical, the reaction is carried out between about 0°C and about 30°C. When W is alkoxy, the reaction is preferably carried out between about 100°C and about 150°C. When W is methyl sulfonate, the reaction is preferably carried out between about -20°C to about 20°C. When W is an ester, the reaction is preferably carried out between about 0°C and about 50°C.

Preparation of the compounds of the present invention by Process C is preferably carried out at about atmospheric pressure, although higher or lower pressures can be used if desired.

Substantially equimolar amounts of reactants are preferably used in Process C, although higher or lower amounts can be used if desired.

Generally, about one equivalent of base is used per equivalent of the reactant of Formula V.

The compounds of Formula II can be prepared by procedures well known to those skilled in the art. By way of example, a suitably substituted hydrazine (such as t-butylhydrazine) is reacted with a heterocyclic ester

(such as ethyl 2-pyridylcarboxylate) in an inert or substantially inert solvent or mixture of solvents (such as ethanol), with heat, to afford the compounds of Formula II (such as N′-t-butyl-N-(2-pyridylcarbonyl)hydrazine; a heterocycle carboxylic acid (such as 2-carboxypyrazine) is reacted with methanesulfonyl chloride in the presence of a base (such as triethylamine) in an inert or substantially inert solvent or mixture of solvents (such as methylene chloride) to afford the corresponding mixed anhydride (such as pyrazine carboxylic-methanesulfonic anhydride which is then reacted with a suitably substituted hydrazine (such as t-butylhydrazine) in the presence of a base (such as triethylamine) in an inert or substantially inert solvent or mixture of solvents (such as methylene chloride) to afford the compounds of Formula II (such as N′-t-butyl-N-(2-pyrazinecarbonyl)hydrazine); a suitably substituted hydrazine (such as t-butylhydrazine) is reacted with a heterocyclic carboxylic acid halide (such as 3-pyridinecarboxylic acid chloride) in the presence of a base (such as sodium hydroxide) in an inert or substantially inert solvent or mixture of solvents (such as toluene) to afford the compounds of Formula II (such as N′-t-butyl-N-(3-pyridinecarbonyl)hydrazine.

Suitably substituted hydrazines such as t-butylhydrazine, isopropylhydrazine and the like are commercially available or can be prepared by procedures well known to those skilled in the art.

The compounds of Formula IV can be prepared from commercially available materials by procedures known to those skilled in the art. By way of example, a suitably substituted hydrazine (such as t-butylhydrazine) is reacted with a benzoyl chloride (such as benzoyl chloride, 3-methylbenzoyl chloride or 4-chlorobenzoyl chloride) in the presence of a base (such as aqueous sodium hydroxide) in an inert or substantially inert solvent or mixture of solvents (such as toluene) to afford the compounds of Formula IV (such as N′-t-butyl-N-benzoylhydrazine, N′-t-butyl-N-(3-methylbenzoyl)hydrazine or N′-t-butyl-(4-chlorobenzoyl)hydrazine).

The compounds of Formula V are commercially available, such as nicotinoyl chloride hydrochloride, isonicotinoyl chloride hydrochloride and ethyl picolinate or can be prepared from commercially available materials by procedures known to those skilled in the art as described above.

The compounds of Formula VII can be prepared by procedures known to those skilled in the art from commercially available reactants. By way of example, a suitably substituted hydrazine (such as t-butylhydrazine) is reacted with an aldehyde or ketone (such as acetone) in the presence of a base (such as triethylamine) to afford a hydrazone which is then reacted with a benzoyl chloride in an inert or substantially inert solvent or mixture of solvents in the presence of a base (such as sodium hydroxide) to afford an N′-substituted-N′-benzoylhydrazone which is then reacted with an acid (such as hydrochloric acid) to afford the compound of Formula VII. Alternatively, a suitably substituted hydrazine (such as t-butylhydrazine) is reacted with di-tert-butyldicarbonate in an inert or substantially inert solvent or mixture of solvents (such as toluene/water) to afford an N′-t-butyl-N-t-butoxycarbonylhydrazine which is then reacted with a benzoylchloride in an inert or substantially inert solvent or mixture of solvents to afford an N′-t-butyl-N′-benzoyl-N-t-butoxycarbonylhydrazine which is then reacted with an acid to afford the desired compound of Formula VII.

Modifications to the above processes may be necessary to accommodate reactive functionalities of particular A and/or B substituents. Such modifications would be apparent and known to those skilled in the art.

It will be appreciated by those skilled in the art that electronic attractive forces may give rise to more than one isomer of the compounds of Formula I. There may be a difference in properties such as biological activity and physical characteristics between such isomers. It is believed the procedures for making the compounds of Formula I described herein will not preferentially afford one isomer over another. Separation of a specific isomer can be accomplished by standard techniques well known to those skilled in the art such as silica gel chromatography.

The agronomically acceptable salts embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide or nitrate with a metal salt of a compound of Formula I in a suitable solvent. When metal hydroxides are used as reagents, useful solvents include water; ethers such as glyme; dioxane; tetrahydrofuran; alcohols such as methanol, ethanol and isopropanol. When metal hydrides are used as reagents, useful solvents include nonhydroxylic solvents, for example, ethers such as dioxane, glyme and diethylether; tetrahydrofuran; hydrocarbons such as toluene, xylene, hexane, pentane, heptane and octane; dimethylformamide. When amines are used as reagents, useful solvents include alcohols, such as methanol or ethanol; hydrocarbons, such as toluene, xylene and hexane; tetrahydrofuran; glyme; dioxane; or water. When ammonium salts are used as reagents, useful solvents include water; alcohols, such as methanol or ethanol; glyme; tetrahydrofuran. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0°C to about 100°C, preferably at about

room temperature.

The acid addition salts of the present invention can be prepared by reacting hydrochloric, hydrobromic, sulfuric, nitric, phosphoric, acetic, propionic, benzoic or other suitable acid with a compound of Formule I having a basic functional group in a suitable solvent. Useful solvents include water, alcohols, ethers, esters and ketones haloalkanes. The particular choice of solvent will depend on the relative solubilities of the starting materials and the resulting salts and slurries rather than solutions of certain reagents may be used to obtain the salts. Generally, equivalent molar amounts of starting materials are used and the salt-forming reaction is carried out at from about -10°C to about 100°C, preferably at about room temperature.

The following examples will further illustrate this invention. In Table I, six-membered heterocyclic derivatives of some N'-substituted-N,N'-diacyl hydrazines of the present invention that have been made are listed. The structure of these compounds was confirmed by NMR and in some cases by IR and/or elemental analysis. Specific illustrative preparation of the compounds of Examples 1, 2, 3, 6, 8, 12, 16, 17, 18 and, 33A and 33B are described after Table I. Examples 7 and 27 are Comparative.

TABLE I

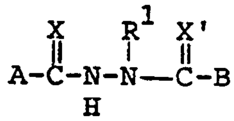

| Ex. No. | X | X' | $R^1$ | A | B | m.p. °C |
|---|---|---|---|---|---|---|
| 1 | O | O | $-C(CH_3)_3$ | $C_6H_5$ | 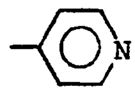 | >210 |
| 2 | O | O | $-C(CH_3)_3$ | 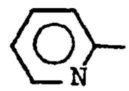 | $C_6H_5$ | >210 |
| 3 | O | O | $-C(CH_3)_3$ | $C_6H_5$ | 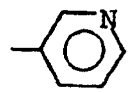 | 60-63 |
| 4 | O | O | $-C(CH_3)_3$ | 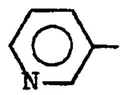 | $-C_6H_3Cl_2$-3,4 | 231-233 |
| 5 | O | O | $-C(CH_3)_3$ | 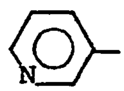 | $C_6H_5$ | 171-172 |
| 6 | O | O | $-C(CH_3)_3$ | 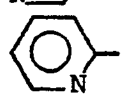 | $-C_6H_4NO_2$-2 | 137-140 |
| 7* | O | O | $-C(CH_3)_3$ | 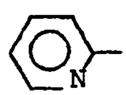 | $-C_6H_4CH_2CH_3$-4 | 207-208 |
| 8 | O | O | $-C(CH_3)_3$ | 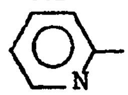 | $-C_6H_4Br$-2 | 179-180 |
| 9 | O | O | $-C(CH_3)_3$ | $-C_6H_4CH_2CH_3$-4 | 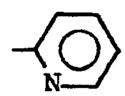 | 129-133 |
| 10 | O | O | $-C(CH_3)_3$ | $-C_6H_4OCF_3$-4 | 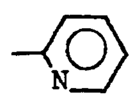 | 140-145 |
| 11 | O | O | $-C(CH_3)_3$ | 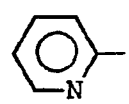 | $-C_6H_4CH_3$-3 | 174-175 |
| 12 | O | O | $-C(CH_3)_3$ | 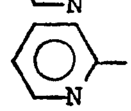 | $-C_6H_3Cl_2$-3,4 | 175-178 |
| 13 | O | O | $-C(CH_3)_3$ | $CH_3\ (CH_2)_3$ 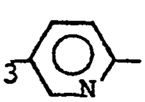 | $C_6H_5$ | 135-140 |

| Ex. No. | X | X' | $R^1$ | A | B | m.p. °C |
|---|---|---|---|---|---|---|
| 14 | O | O | $-C(CH_3)_3$ | $C_6H_5$ | 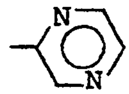 | 135-140 |
| 15 | O | O | $-C(CH_3)_3$ | 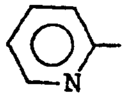 | $-C_6H_3(CH_3)_2$-3,5 | 162-164 |
| 16 | O | O | $-C(CH_3)_3$ | 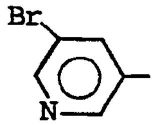 | $-C_6H_4Cl$-4 | 193-197 |
| 17 | O | O | $-C(CH_3)_3$ | 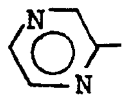 | $C_6H_5$ | 181-183 |
| 18 | O | O | $-C(CH_3)_3$ | 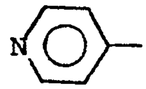 | $C_6H_5$ | 196-198 |
| 19 | O | O | $-C(CH_3)_3$ | 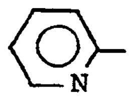 | $-C_6H_4OCH_3$-4 | 208-210 |
| 20 | O | O | $-C(CH_3)_3$ | 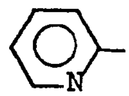 | $-C_6H_4I$-2 | 175 |
| 21 | O | O | $-C(CH_3)_3$ | 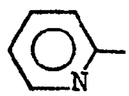 | $-C_6H_3Cl_2$-2,4 | 97-98 |
| 22 | O | O | $-C(CH_3)_3$ | 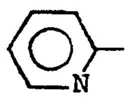 | $-C_6H_4F$-4 | 215-218 |
| 23 | O | O | $-C(CH_3)_3$ | 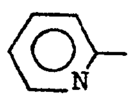 | $-C_6H_4CF_3$-2 | 126-128 |
| 24 | O | O | $-C(CH_3)_3$ | 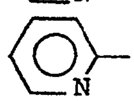 | $-C_6H_4NO_2$-3 | 176-179 |
| 25 | O | O | $-C(CH_3)_3$ | 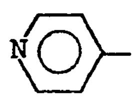 | $C_6H_4CH_3$-3 | 203-206 |
| 26 | O | O | $-C(CH_3)_3$ | 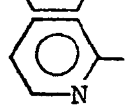 | $C_6H_4Cl$-2 | 160-162 |
| 27* | O | O | $-C(CH_3)_3$ | 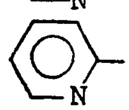 | $C_6H_4OCH_3$-3 | 225-228 |
| 28 | O | O | $-C(CH_3)_3$ | 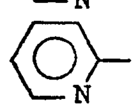 | $C_6H_4F$-4 | 201-205 |
| 29 | O | O | $-C(CH_3)_3$ | $C_6H_5$ | 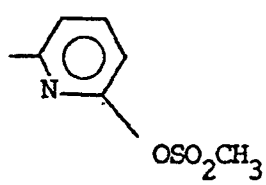 | 176-178 |

| Ex. No. | X | X' | $R^1$ | A | B | m.p. °C |
|---|---|---|---|---|---|---|
| 30 | O | O | $-C(CH_3)_3$ | 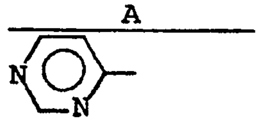 | $C_6H_4F$-4 | >250 |
| 31 | O | O | $-C(CH_3)_3$ | $C_6H_5$ | 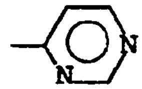 | 188-190 |
| 32 | O | O | $-C(CH_3)_3$ | 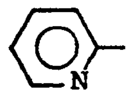 | $C_6H_3(CH_3)_2$-2,3 | 157-158 |
| 33A | O | O | $-C(CH_3)_3$ | $C_6H_5$ | 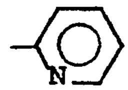 | 220 |
| 33B | O | O | $-C(CH_3)_3$ | $C_6H_5$ | 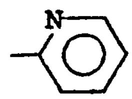 | 173 |
| 34 | O | O | $-C(CH_3)_3$ | 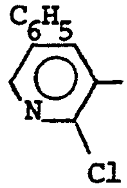 | $C_6H_4CH_3$-3 | 200-202 |
| 35 | O | O | $-C(CH_3)_3$ | $C_6H_3(CH_3)_2$-2,3 | 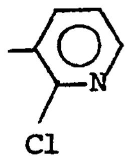 | 190 |
| 36 | O | O | $-C(CH_3)_3$ | 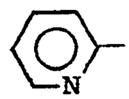 | $C_6H_3ClCH_3$-2,5 | 115 |
| 37 | O | O | $-C(CH_3)_3$ | 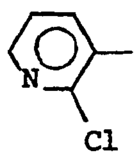 | $C_6H_3ClCH_3$-2,5 | 115-120 |
| 38 | O | O | $-C(CH_3)_3$ | 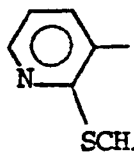 | $C_6H_4CH_3$-3 | 214-219 |
| 39 | O | O | $-C(CH_3)_3$ | 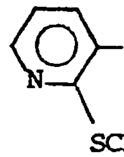 | $C_6H_3Cl_2$-3,4 | >225 |
| 40 | O | O | $-C(CH_3)_3$ | 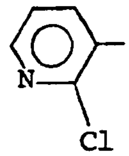 | $C_6H_5$ | 241-243 |
| 41 | O | O | $-C(CH_3)_3$ | 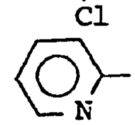 | $C_6H_3ClF$-2,4 | 152-155 |

*Comparative

EXAMPLE 1 - Preparation of N'-t-butyl-N-benzoyl-N'-isonicotinoylhydrazine

N'-t-butyl-N-benzoylhydrazine (1.0 g, 0.0052 mol) was suspended in 20 ml of toluene. Isonicotinoyl chloride hydrochloride (0.93 g, 0.0052 mol) was added and then a solution of sodium hydroxide (1.25 g of 50% aqueous NaOH) in 5 ml of water was added dropwise. After stirring at 23°C for 2 hours, the solids were removed by

filtration, washed with water and dried in air. The crude product was chromatographed on silica gel using 5% methanol/methylene chloride as eluant to afford pure N'-t-butyl-N-benzoyl-N'-isonicotinoylhydrazine. m.p. >210°C.

EXAMPLE 2 - Preparation of N'-t-butyl-N-(2-pyridinecarbonyl)-N'-benzoylhydrazine

A solution of N'-t-butyl-N-(2-pyridinecarbonyl)hydrazine (1.0 g, 0.00518 mol) in 20 ml of toluene at 23°C was treated sequentially with 50% sodium hydroxide (1.3 g) and benzoyl chloride (0.728 g). The mixture was stirred overnight. The solids were removed by filtration and washed with water to afford N'-t-butyl-N-(2-pyridinecarbonyl)-N'-benzoylhydrazine.

EXAMPLE 3 - Preparation of N'-t-butyl-N-benzoyl-N'-nicotinoylhydrazine

A solution of N'-t-butyl-N-benzoylhydrazine (2.0 g) and nicotinoyl chloride hydrochloride in 20 ml of methylene chloride at 23°C was treated dropwise with triethylamine (4 ml). The reaction mixture was stirred at 23°C for 0.5 hours. Solids were removed by filtration. The filtrate was diluted with N/10 HCl and ether. The layers were separated and the organic layer was washed with N/10 HCl. The aqueous layer was neutralized with solid sodium bicarbonate and extracted with ether. The ether extracts were treated with charcoal and then dried with magnesium sulfate. Evaporation of solvents afforded a yellow oil which was chromatographed on silica gel using 10% $CH_3OH$, 40% $CH_2Cl_2$, 50% $Et_2O$ as eluant to afford N'-t-butyl-N-benzoyl-N'-nicotinoylhydrazine as a yellow foam. m.p. 60-63°C.

EXAMPLE 6 - Preparation of N'-t-butyl-N-(2-pyridinecarbonyl)-N'-(2-nitrobenzoyl)hydrazine

A solution of N'-t-butyl-N-(2-pyridinecarbonyl)hydrazine (1.0 g, 0.00518 mol) in 20 ml of toluene was treated dropwise simultaneously with a solution of sodium hydroxide (1.24 g of 50% aqueous solution) in 5 ml of water and 2-nitrobenzoyl chloride (0.96 g). The resulting mixture was stirred at 23°C overnight. Water was added and the mixture was extracted with ether. A second extraction was performed with methylene chloride and the combined organic extracts were dried over magnesium sulfate. Evaporation afforded 0.4 g of N'-t-butyl-N-(2-pyridinecarbonyl)-N'-(2-nitrobenzoyl)hydiazine as a yellow solid. m.p. 137-140°C.

EXAMPLE 8 - Preparation of N'-t-butyl-N-(2-pyridinecarbonyl)-N'-(2-bromobenzoyl)hydrazine

An aqueous solution of sodium hydroxide (1.24 g of 50% NaOH diluted with 5 ml of water) was added to a solution of N'-t-butyl-N-(2-pyridinecarbonyl)hydrazine (1.0 g, 0.00518 mol) in 20 ml of toluene at 23°C. The mixture was cooled and treated with 2-bromobenzoyl chloride (1.137 g, 0.00518 mol). The mixture was then stirred at 23°C overnight. The solids were removed by filtration, washed with water and dried to afford 0.96 g of N'-t-butyl-N-(2-pyridinecarbonyl)-N'-(2-bromobenzoyl)hydrazine as a white solid. m.p. 179-180°C.

EXAMPLE 12 - Preparation of N'-t-butyl-N-(2-pyridinecarbonyl)-N'-(3,4-dichlorobenzoyl)hydrazine

A solution of N'-t-butyl-N-(2-pyridinecarbonyl)hydrazine (0.5 g) in 10 ml of toluene was treated with 50% sodium hydroxide (0.61 g). 3,4-dichlorobenzoyl chloride (0.6 g) was added and the mixture was stirred rapidly for 4 hours at 23°C and then was allowed to stand for 48 hours. The solids were removed by filtration and washed with water to afford. 75 g of N'-t-butyl-N-(2-pyridinecarbonyl)-N'-(3,4-dichlorobenzoyl)hydrazine as a white solid. m.p. 175-178°C.

EXAMPLE 16 - Preparation of N'-t-butyl-N-(5-bromonicotinoyl)-N'-(4-chlorobenzoyl)hydrazine

N'-t-butyl-N'-(4-chlorobenzoyl)hydrazine was prepared substantially according to the procedured for preparing N'-t-butyl-N'-benzoylhydrazine described for Example 18 except 4-chlorobenzoyl chloride was used in place of benzoyl chloride.

A solution of N'-t-butyl-N'-(4-chlorobenzoyl)hydrazine (0.5 g, 0.0022 mol) and 5-bromonicotinic acid (0.44 g, 0.0022 mol) in 10 ml of methylene chloride containing triethylamine (0.33 g) was added to a solution of methanesulfonyl chloride (0.25 g, 0.0022 mol) in 10 ml of methylene chloride at 0°C. The resulting mixture was stirred at 23°C for 2 hours and then was allowed to stand overnight at 23°C. Aqueous sodium bicarbonate was added and the layers were separated. The aqueous layer was re-extracted with methylene chloride. The organic extracts were evaporated to give a yellow solid which was triturated with hexane/methylene chloride to afford

N'-t-butyl-N-(5-bromonicotinoyl)-N'-(4-chlorobenzoyl)hydrazine as an off-white solid. m.p. 193-197°C.

EXAMPLE 17 - Preparation of N'-t-butyl-N-(pyrazinecarbonyl)-N'-benzoylhydrazine

To a mechanically stirred solution of t-butylhydrazine hydrochloride (51 g, 0.41 mol) in dioxane (100 ml) and water (50 ml), cooled in an ice bath was treated with 50% sodium hydroxide (32g). The resulting mixture was treated dropwise with di-t-butyldicarbonate (92g, 0.42 mol) over about one-half of an hour. After complete addition, the reaction mixture was warmed to room temperature and stirred for 2 hours. The resulting white solid was filtered off, washed with water and air-dried to afford 74g of N'-t-butyl-N-t-butoxycarbonylhydrazine, m.p. 69-71°C.

A mechanically stirred solution of N'-t-butyl-N-t-butoxycarbonylhydrazine (61g, 0.32 mol) in toluene (120 ml) cooled in an ice bath, was treated dropwise and simultaneously with 50% sodium hydroxide (31g) in water (50 ml) and benzoyl chloride (45g). The addition was complete in 20 minutes and the resulting mixture was warmed to room temperature and allowed to stir for one hour. The resulting white solid was filtered, washed with water and air dried to afford 94g of N-t-butoxycarbonyl-N'-benzoyl-N'-t-butylhydrazine, mp. 167-170°C.

To a mechanically stirred solution of N-t-butoxycarbonyl-N'-benzoyl-N'-t-butylhydrazine (52g, 0.18 mol) in methanol (100 ml) was added concentrated hydrochloric acid (35 ml). The resulting mixture was stirred at room temperature for 4 days and then neutralized with saturated aqueous sodium bicarbonate. The resulting white solid was filtered, washed with water and dried under vacuum to afford N'-t-butyl-N'-benzoylhydrazine (39g) mp. 124-125°C.

Triethylamine (1.0 g, 0.01 mol) was added to a solution of N'-t-butyl-N'-benzoylhydrazine (0.86 g, 0.0031 mol) and pyrazine carboxylic acid (0.56 g, 0.0045 mol) in 10 ml of methylene chloride at 23°C. This mixture was added to a solution of methanesulfonyl chloride (0.6 g, 0.0052 mol) in 10 ml of methylene chloride at 0°C. The mixture was stirred at 23°C for 3 hours and then allowed to stand at 23°C overnight. Aqueous sodium bicarbonate was added and the layers were separated. The organic extracts were evaporated to give crude product which was triturated with ether to afford N'-t-butyl-N-(pyrazinecarbonyl)-N'-benzoylhydrazine as a white solid. m.p. 181-183°C.

EXAMPLE 18 - Preparation of N'-t-butyl-N-isonicotinoyl-N'-benzoylhydrazine

A solution of N'-t-butyl-N'-benzoylhydrazine (0.5 g, 0.00026 mol) in 10 ml of toluene was treated with 50% sodium hydroxide (0.6 g) followed by isonicotinyl chloride hydrochloride (0.47 g, 0.0026 mol). The mixture was stirred at 23°C overnight. The solids were removed by filtration and washed with water followed by ether to afford N'-t-butyl-N'-isonicotinoyl- N'-benzoylhydrazine.

EXAMPLE 22 - Preparation of N'-t-butyl-N-(2-pyridinecarbonyl)-N'-(4-fluorobenzoyl)hydrazine

By substantially following the procedures described above for Example 2, except using 4-fluorobenzoyl chloride rather than benzoyl chloride, N'-t-butyl-N-(2-pyridinecarbonyl)-N'-(4-fluorobenzoyl)hydrazine was afforded.

EXAMPLES 33A and 33B - Preparation of N'-t-butyl-N'-(2-pyridinecarbonyl)-N-benzoylhydrazine

To a suspension of 2-picolinic acid (12.8g, 0.104 mol) in methylene chloride (80 ml) were added dropwise triethylamine (14g, 0.139 mol) in 10 ml methylene chloride followed by methanesulfonyl chloride (13g, 0.113 mol) in 10 ml methylene chloride at 0°C. The resulting mixture was stirred for half an hour before the dropwise addition of N'-t-butyl-N-benzoylhydrazine (20.0g, 0.10 mole) in 80 ml methylene chloride at 0°C to 23°C. The final dark brown mixture was allowed to stir at 23°C for one hour, and to stand at 23°C overnight.

Aqueous sodium bicarbonate was added and the layers were separated. The aqueous layer was re-extracted with methylene chloride. The organic extracts were combined, and dried over magnesium sulfate. Evaporation under reduced pressure gave 25 g of a light green, brown solid. Recrystallization on a steam bath with ethyl acetate:hexane (80:20v/v) afforded a light yellow solid.

Column chromatography on silica gel, eluted first with methylene chloride and ether and then with ethyl acetate afforded two separate isomers of N'-t-butyl-N'-(2-pyridinecarbonyl)-N-benzoylhydrazine.

By following substantially the procedures in the processes described above and as exemplified by the preparation of the compounds of Examples, 1, 2, 3, 6, 8, 12, 16, 18, and 33A and 33B, the compounds of Formula I are prepared.

As previously noted, the compounds of the present invention exhibit excellent insecticidal activity and are

selective against insects of the order Lepidoptera.

In general, for the control of insects in agriculture, horticulture and forestry, the compounds of the present invention may be used at a dosage corresponding to from about 10 grams to about 10 kilograms of the active substance per hectare and from about 100 grams to about 5 kilograms per hectare of the active substance is preferred. The exact amount of dosage for a given situation can be routinely determined and depends on a variety of factors, for example, the substance used, the kind of insect, the formulation used, the state of the crop infested with the insect and the prevailing weather conditions. The term "insecticidal" as employed in the specification and claims of this application is to be construed as any means which adversely affects the existence or growth of the target insects. Such means can comprise a complete killing action, eradication, arresting in growth, inhibition, reducing in number or any combination thereof. The terms "control" and "combat" as employed in the specification and claims of this application are to be construed as including "insecticidal" and the protection of plants from insect damage. By "insecticidally effective amount" is meant that dosage of active substance sufficient to exert insect "control" or to "combat" insects.

The compounds of the present invention, for practical applications, can be utilized in the form of compositions or formulations. Examples of the preparation of compositions and formulations can be found in the American Chemical Society publication "Pesticidal Formulation Research", (1969), Advances in Chemistry Series No. 86, written by Wade Van Valkenburg; and the Marcel Dekker, Inc. publication "Pesticide Formulations", (1973), edited by Wade Van Valkenburg. In these compositions and formulations, the active substance or substances are mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, conventional adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behavior, feeding habits and physiology of the pest for the purpose of control.

The invert emulsions are mainly used for air application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene toluene, xylene, alkyl naphthalenes), halogenated, especially chlorinated, aromatic hydrocarbons (e.g., chlorobenzenes), cycloalkanes (e.g., cyclohexane), paraffins (e.g., petroleum or mineral oil fractions), chlorinated aliphatic hydrocarbons (e.g., methylene chloride, chloroethylenes), vegetable oils (e.g., soybean oil, cottonseed oil, corn oil), alcohols (e.g., methanol, ethanol, propanol, butanol, glycol) as well as ethers and esters thereof (e.g., glycol monomethyl ether), amines (e.g., ethanolamine), amides (e.g., dimethyl formamide), sulfoxides (e.g., dimethyl sulfoxide,), acetonitrile, ketones (e.g., acetone, methyl ethyl ketone, methyl isobutyl ketone, cyclohexanone, isophorone), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, and especially alkyl arylpolyglycol ethers, magnesium stearate, sodium oleate); and/or dispersing agents, such as lignin, sulfite waste liquors, methyl cellulose.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible diluent or carrier vehicles and/or with other known compatible active agents, especially plant protection agents, such as other insecticides, arthropodicides, nematicides, fungicides, bactericides, rodenticides, herbicides, fertilizers, growth-regulating agents, synergists, if desired, or in the form of particular dosage preparations for specific application made thereform, such as solutions, emulsions, suspensions, powders, pastes, and granules which are thus ready for use.

As concerns commercially marketed preparations, these generally contemplate carrier composition mixtures in which the active compound is present in an amount substantially between about 0.1% and 99% by weight, preferably between about 0.1% and 90% by weight, and more preferably between about 1% and 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally, between about 0.0001% and about 99% by weight of the composition, preferably between about 0.001% and about 90% by weight of the composition, and more preferably between about 0.01% and about 75% by weight of the composition, which is effective for the purpose in question.

The active compounds can be applied as sprays by methods commonly employed, such as conventional high-gallonage hydraulic sprays, low gallonage sprays, ultra-low-volume sprays, airblast spray, aerial sprays, and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 micrometres or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of killing, combatting or controlling insects, which comprises contacting insects with a correspondingly combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects and (b) the corresponding habitat thereof (i.e., the locus to be protected, for example, to a growing crop or to an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example, as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke, are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

Representative preparation of compositions and formulations including the compounds of the present invention are set forth below as Examples A through I by way of illustration but not limitation.

Example A

Granular

| Ingredient | %/wt. |
| --- | --- |
| Toxicant and toxicant impurities | 0.25 |
| Triton X-305 (binder) (Octylphenyl-30-ethylene oxide ethanol) | 0.25 |
| Agsorb 24/48 (diluent) (Montmorillonite clay) | 99.50 |

Preparation: The toxicant and Triton X-305 are dissolved into methylene chloride and the mixture is added to the Agsorb with continuous mixing. The methylene chloride is then allowed to evaporate.

Example B

Dust

| Ingredient | %/wt. |
| --- | --- |
| Toxicant and toxicant impurities | 1.0 |
| Talc | 99.0 |

Preparation: Toxicant is dissolved in excess acetone and the mixture is impregnated onto the talc. The acetone is then permitted to evaporate.

Example C

Wettable Powder

| Ingredient | %/wt. |
| --- | --- |
| Toxicant and toxicant impurities | 31.3 |
| Duponal WA Dry (wetter) (Sodium lauryl sulfate) | 2.0 |
| Reax 45A (dispersant) (Sodium lignin sulfonate) | 5.0 |
| Barden clay (diluent) | 31.7 |
| HiSil 233 (diluent) (Sodium silica) | 30.0 |

Preparation: The toxicant, optionally dissolved in a volatile solvent, is absorbed onto the Barden clay and HiSil carriers. The Duponal and Reax are then added and the entire dry mixture blended until homogeneous. The composition is then micronized to a fine particle size.

Example D

Emulsifiable Concentrate

| Ingredient | %/wt. |
|---|---|
| Toxicant and toxicant impurities | 15.0 |
| Sponto 232T (emulsifier) (Anionic and nonionic blend of the following surfactants: calcium dodecyl benzene sulfonate; and ethoxylated alkylphenol) | 6.0 |
| Sponto 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco 500-100 (solvent) (Aromatic solvent mixture principally comprising xylene, cumene and ethyl benzene having a boiling point range of 290-345°F) | 52.5 |

Preparation: All ingredients are mixed together with continuous agitation until a homogeneous clear solution is obtained.

Example E

Aerosol

| Ingredient | %/wt. |
|---|---|
| Toxicant and toxicant impurities | 0.5 |
| Freon 12 | 99.5 |

Preparation: The components are mixed and packaged under pressure in a suitable container equipped with a release spray valve.

Example F

Fumigating Candle or Fumigating Powder

| Ingredient | %/wt. |
|---|---|
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon 4200 (preservative) (2-*n*-octyl-4-isothiazolin-3-one) | 0.05 |

Preparation: The corn oil and corn syrup are added to the wheat bran with adequate mixing. The toxicant and Kathon are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example H

Pellet

Same as Example G, Method A, with this addition: the bait composition is formed into 0.635 cm (1/4") diameter by 0.95 cm (3/8") long pellets using a suitable die and press apparatus.

Example I

Flowable

| Ingredient | %/wt. |
|---|---|
| Toxicant and toxicant impurities | 31.3 |
| Duponal WA Dry (wetter) | 2.0 |
| (Sodium lauryl sulfate) | |
| Reax 45A (dispersant) | 5.0 |
| (Sodium lignin sulfonate) | |
| HiSil 233 (diluent) | 30.0 |
| (Sodium silica) | |
| Kelzan (thickener) | 0.5 |
| (Xanthan gum) | |
| Water | 31.2 |

Preparation: The toxicant is absorbed onto the HiSil carrier. The Duponal and Reax are then added and the entire dry mixture blended until homogeneous. The composition is then micronized to a fine particle size. The resulting powder is suspended in water and the Kelzan added.

Compositions ant formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis(p-chlorophenyl)-1,1,1-trichloroethane and hexachloroepoxyoctahydrodimethanonaphthalene;

Carbamates, for example, N-methyl-1-naphthylcarbamates;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, O,O-diethyl-O-p-nitrophenylphosphorothioate; N-monomethylamide or O,O-dimethyldithiophosphorylacetic acid;

Diphenylsulfides, for example, p-chlorobenzyl or p-chlorophenyl sulfide and 2,4,4',5-tetrachlorodiphenylsulfide;

Diphenylsulfonates, for example, p-chlorophenylbenzenesulfonate;

Methylcarbinols, for example, 4,4-dichloro-1-trichloromethylbenzhydrol;

Quinoxaline compounds, such as methylquinoxaline dithiocarbonate;

Amidines such as N'-(4-chloro-2-methylphenyl) N,N-dimethylformamidine;

Pyrethroids such as Allethrin;

Biologicals such as Bacillus thuringiensis preparations;

Organic tin compounds such as tricyclohexyltin hydroxide;

Synergists such as piperonyl butoxide.

Fungicides such as:

Organic mercury compounds, for example, phenylmercuryacetate and methylmercurycyanoguanide;

Organic tin compounds, for example, triphenyltin hydroxide and triphenyltin acetate;

Alkylenebisdithiocarbamates, for example, zinc ethylenebisthiocarbamate and manganese ethylenebisdithiocarbamate; and

2,4-dinitro-6-(2-octyl-phenylcrotonate), 1-bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrile, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile.

Biological Activity

It has been found by biological evaluation that compounds according to the present invention have pesticidal activity and are capable of controlling larvae and adult forms of pests, especially insects from the order Lepidoptera. One skilled in the art will know how to determine the activity of a given compound against a given insect and the dosage required to obtain general or selective insecticidal effects. The compounds of the present invention in part affect the normal development of insects, particularly insects from the order Lepidoptera, by directly and/or indirectly influencing the moulting process.

As previously noted, the compounds of the present invention are particularly suitable for controlling plant destructive insects in crops of cultivated plants, such as, but not limited to, cotton, vegetables, corn and other cereals and the like; forestry, such as, but not limited to, birch, spruce, pine, fir and the like; and ornamental plants, flowers and trees. Compounds of the present invention are also particularly suitable for controlling insects destructive to stored commodities such as seeds and the like; fruit crops, such as, but not limited to fruit and/or citrus trees, raspberry bushes and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton X-155) and a modified phthalic glycerol alkyl resin (sold under the trademark Triton B-1956) was utilized at the equivalent of 28.35 g (1 ounce) per 378.5 1 (100 gal) of test solution as a surfactant.

Initial evaluations were made on one or more of the following pests:

| Code Symbol | Common Name | Latin Name |
|---|---|---|
| SAW | Southern Armyworm | Spodoptera eridania |
| MBB | Mexican Bean Beetle | Epilachna varivestis |
| BW | Boll Weevil | Anthonomus grandis grandis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

For the Boll Weevil test ten adult weevils are placed in a 0.237 1 (0.5 pint) glass Mason jar containing a small cube of apple. The weevils are confined to the jars by fiberglass screen mesh secured by a screw-type rim cap. The jars are then sprayed with the test solution using a rotating turntable, directing the spray through the mesh onto the jar.

Percent mortalities for the bean beetle and armyworm evaluations are determined 96 hours after treatment. Boll weevil mortality is determined 48 hours after treatment. Evaluations are based on a scale of 0-100 percent in which 0 equals no activity and 100 equals total kill.

The rotating turntable consists of a fixed, continuously operating spray nozzle under which targets are rotated at a fixed speed and distance. If the target is a Petri dish (such as for the armyworm), the distance from the nozzle is 38.1 cm (15 inches). If the target is a Mason jar, the distance between the screened lid and the nozzle is 15.24 cm (6 inches) (25.4 cm (10 inches) from the base of the jar to the nozzle). The nozzle is located 20.32 cm (8 inches) from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At a guage pressure of $6.85 \times 10^4$ $N/m^2$ (10 psig) air pressure used and with liquid siphon feed 1.9 1/h (0.5 gal per hour) are delivered in a round spray pattern with a 21° spray angle. Targets are misted with spray droplets to the point that the droplets coalesce to form a uniform thin film insufficient to drown test organisms.

All treatments are maintained at 23.9-26.7°C (75-80°F) under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 7.62 cm (3 inch) pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control (mortality) is determined. A second observation may be made 6 days after infesting the dishes if the experimenter feels the effect may not be complete or moribund insects appear to evidence signs of some recovery. Where necessary, untreated bean leaves are introduced into dishes held for a second observation to preclude insect starvation.

The results of the initial insecticidal evaluations are given in Table II.

Armyworm and bean beetle spray (foliar) results are 96 hour observations. Boll Weevil spray results are 48 hour observations. Soil treatment results are 72 hour observations. At the discretion of the experimenter, particular evaluations were held for 144 hour observations. If, after 144 hours, there was a change in the percent control, it is shown in parentheses.

## TABLE II

### Initial Biological Evaluations

| Example No. | Foliar Application Test Species SAW | MBB | BW | Soil Application Test Species MBB | SAW |
|---|---|---|---|---|---|
| 1 | 50 | 0 | 40 | 0 | 0 |
| 2 | 100 | 80 | 40 | 60(100) | 90(100) |
| 3 | 90 | 10 | 0 | 0(20) | 0 |
| 4 | 100 | 0 | 0 | 0 | 0 |
| 5 | 100 | 30 | 0 | 20 | 100 |
| 6 | 100 | 30 | 0 | 100 | 100 |
| 7(Comparative) | 0 | 0 | 0 | 0 | 0 |
| 8 | 100 | 70 | 0 | 100 | 100 |
| 9 | 100 | 0 | 0 | 0 | 10 |
| 10 | 70 | 50 | 0 | 40(20) | 50(60) |
| 11 | 100 | 0 | 20 | 0 | 100 |
| 12 | 100 | 50 | 0 | 0 | 80(100) |
| 13 | 0 | 0 | 20 | 0 | 0 |
| 14 | 100 | 10 | 0 | 20 | 80(100) |
| 15 | 100 | 0 | 0 | 0 | 90(100) |
| 16 | 100 | 0 | 0 | 0 | 0 |
| 17 | 100 | 80 | 0 | 80 | 100 |
| 18 | 100 | 20 | 0 | 0 | 0 |
| 19 | 30 | 30 | 0 | 0 | 0 |
| 20 | 100 | 0 | 0 | n.t. | 0 |
| 21 | 100 | 0 | 0 | n.t. | 0 |
| 22 | 100 | 40 | 0 | n.t. | 0 |
| 23 | 100 | 20 | 0 | n.t. | 0 |
| 24 | 100 | 10 | 0 | n.t. | 0 |

| | | | | | |
|---|---|---|---|---|---|
| 25 | 100 | 0 | 0 | n.t. | 0 |
| 26 | 70 | 20 | 0 | n.t. | 0 |
| 27 (Comparative) | 0 | 0 | 0 | n.t. | 0 |
| 28 | 100 | 0 | 0 | n.t. | 0 |
| 29 | 20 | 30 | 0 | n.t. | 0 |
| 30 | 100 | 0 | 20 | n.t. | 100 |
| 31 | 10 | 20 | 0 | n.t. | 0 |
| 32 | 100 | 20 | 0 | n.t. | 0 |
| 33A | 100 | 70 | 0 | n.t. | 100 |
| 33B | 100 | 10 | 0 | n.t. | 0 |
| 34 | 100 | 0 | 0 | n.t. | n.t. |
| 35 | 100 | 0 | 0 | n.t. | n.t. |
| 36 | 100 | 100 | 0 | n.t. | n.t. |
| 37 | 100 | 10 | 0 | n.t. | n.t. |
| 38 | 0 | 40 | 20 (40) | n.t. | n.t. |
| 39 | 0 | 90 | 0 | n.t. | n.t. |
| 40 | 100 | 40 | 0 | n.t. | n.t. |
| 41 | 100 | 90 | 0 | n.t. | n.t. |

a - 72 hours exposure data. Supplemental 144 hour exposure values, where obtained and different, are given in parenthesis.

n.t. = not tested.

In its mechanical aspects therefore a process of the invention for improving the commercial value and/or profitability of vendible crops from plants whose growth is affected or likely to be affected by insects comprises (1) charging to a container, fumigation device or mechanical dissemination device an insecticidal composition of the invention as hereinbefore described (2) using the container, fumigator or mechanical dissemination device to apply the insecticidal composition, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

The following words are trademarks which may be registered in some or all of the designated states: Triton, Agsorb, Duponal, Reax, Hisil, Sponto, Tenneco, Kathon and Kelzan.

## Claims

**Claims for the following Contracting States: BE, CH, DE, FR, GB, GR, IT, LI, LU, NL, SE**

1. An insecticidally active compound which is an N'-substituted-N,N'-diacyl hydrazine having the formula

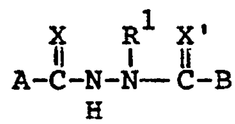

wherein

X and X′ are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitro; cyano; hydroxy; ($C_1$-$C_6$)alkyl, halo-($C_1$-$C_6$)alkyl; cyano-($C_1$-$C_6$)alkyl; ($C_1$-$C_6$)alkoxy; halo-($C_1$-$C_6$)alkoxy; ($C_1$-$C_6$)alkoxy-($C_1$-$C_6$)alkyl having independently the stated number of carbon atoms in each alkyl group; ($C_1$-$C_6$)alkoxy-($C_1$-$C_6$)alkoxy having independently the stated number of carbon atoms in each alkyl group; $-OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; carboxy; $-ZCO_2R'$ group; -COR group; halo-($C_1$-$C_6$)alkyl-carbonyl; cyano-($C_1$-$C_6$)alkyl-carbonyl; ($C_1$-$C_6$)-nitro-($C_1$-$C_6$)alkyl-carbonyl; ($C_1$-$C_6$)alkoxy-carbonyl; halo-($C_1$-$C_6$)alkoxy-carbonyl; -OCOR group; $NH_2$; NHZ; NZZ′; amino substituted with hydroxy, ($C_1$-$C_4$)alkoxy or ($C_1$-$C_4$)alkylthio groups; -CONRR′ group; -OCONRR′ group; -NRCOR′ group; $-NRCO_2R'$ group; -OCONRCOR′ group; sulfhydryl; ($C_1$-$C_6$)alkylthio; halo-($C_1$-$C_6$)alkylthio; -NRCSR′ group; -SCOR group; unsubstituted or substituted phenyl having one to three of the same or different halo, cyano, nitro, hydroxy, ($C_1$-$C_4$)alkyl, ($C_1$-$C_4$)alkoxy, carboxyl, ($C_1$-$C_4$)alkoxy-carbonyl, ($C_1$-$C_4$)alkanoyloxy or amino; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; thio-($C_1$-$C_6$)alkoxy; carboxy; ($C_1$-$C_6$)alkoxy-carbonyl; $-ZCO_2R'$ group; -CONRR′ group; amino; -NRCOR′ group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl, $NH_2$, NHZ or NZZ′;

where R and R ′ are hydrogen or ($C_1$-$C_6$)alkyl; Z and Z′ are ($C_1$-$C_6$)alkyl; "amino" means NRR′; and one of A or B is an unsubstituted or substituted six-membered heterocycle as defined above; provided that B is not 4-alkyl-substituted phenyl or 3-alkoxy-substituted phenyl when A is 2-pyridyl; or an agronomically acceptable salt thereof.

2. A compound according to claim 1 wherein

X and X′ are O or S;

$R^1$ is branched ($C_3$-$C_8$)alkyl; and

A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$)alkoxy-($C_1$-$C_4$)alkyl having independently the stated number of carbon atoms in each alkyl group; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or

unsubstituted or substituted six-membered heterocycle having one or two nitrogen atoms and 4 to 5 nuclear carbon atoms where the substituents can be one or two of the same or different halo; nitro; ($C_1$-$C_4$)alkyl; ($C_1$-$C_4$)alkoxy; thio-($C_1$-$C_4$)alkoxy; -NDD′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NDD′;

where D and D' are hydrogen or $(C_1-C_4)$alkyl.

3. A compound according to claim 2 wherein

X and X' are O;

$R^1$ is branched $(C_4-C_7)$alkyl; and

A and B are phenyl or substituted phenyl where the substituents can be from one to three of the same or different halo, nitro, $(C_1-C_4)$alkyl, $(C_1-C_4)$alkoxy, or halo-$(C_1-C_4)$alkyl; or

unsubstituted or substituted six-membered heterocycle having one or two nitrogen atoms and four or five nuclear carbon atoms where the substituents can be one or two of the same or different halo, nitro, $(C_1-C_4)$alkyl, $(C_1-C_4)$alkoxy or thio-$(C_1-C_4)$alkoxy.

4. A compound according to claim 3 wherein

X and X' are O;

$R^1$ is t-butyl, neopentyl (2,2-dimethylpropyl) or 1,2,2-trimethylpropyl; and

A and B are phenyl or substituted phenyl where the substituents can be one or two of the same or different chloro, fluoro, bromo, iodo, nitro, methyl, ethyl or trifluoromethyl; or

unsubstituted or substituted pyridyl or 2,5-pyrazinyl where the substituent can be halo, nitro, $(C_1-C_4)$alkyl, $(C_1-C_4)$alkoxy or thio-$(C_1-C_4)$alkoxy.

5. A compound according to claim 4 wherein

X and X' are O;

$R^1$ is t-butyl; and

A and B are phenyl or substituted phenyl where the substituents can be one or two of the same or different chloro, fluoro, bromo, iodo, nitro, methyl, ethyl or trifluoromethyl; or pyridyl or 2,5-pyrazinyl, or pyridyl substituted with halo, $(C_1-C_3)$alkyl or thiomethoxy.

6. A compound according to claim 5 wherein

X and X' are O;

$R^1$ is t-butyl; and

A is 2-pyridyl and B is phenyl, 3-methylphenyl, 4-fluorophenyl or 2-chloro-4-fluorophenyl; or

A is 5-bromo-3-pyridyl and B is 4-chlorophenyl; or

A is 2-chloro-3-pyridyl and B is 3-methylphenyl; or

A is 2,3-dimethylphenyl and B is 2-chloro-3-pyridyl.

7. An insecticidal composition comprising an insecticidally active compound according to any preceding claim and agronomically acceptable diluent or carrier.

8. A composition according to claim 7 containing said compound in an amount of 0.0001 to 99%, preferably 0.001 to about 90% by weight of the composition.

9. A composition according to claim 8 containing said compound in an amount of from 0.01 to 75% by weight of the composition.

10. An insecticidal composition according to claim 7 or 8 (a) additionally containing a dispersing agent, said composition being in the form of a wettable powder, or (b) containing a liquid agronomically acceptable carrier and a dispersing agent, said composition being in the form of a flowable, or (c) in the form of a dust, or (d) additionally containing a binding agent, said composition being in the form of a granule, or (e) additionally containing an attractant agent, said composition being in the form of a bait or (f) additionally containing an emulsifying agent, said composition being in the form of an emulsifiable concentrate.

11. A method of controlling insects which comprises contacting said insects with an effective amount of the insecticidally active compound according to any of claims 1 to 6 optionally in a composition according to any of claims 7 to 10.

12. A method according to claim 11 which comprises applying the compound or composition to growing plants or to an area where plants are to be grown at a rate of from 10 grams to 10 kilograms of the active compound per hectare, preferably from 100 grams to 5 kilograms per hectare.

13. A method according to claim 12 wherein said insects are from the order Lepidoptera.

**Claim for the following Contracting State: AT**

1. An insecticidal composition comprising insecticidally active compound which is an N'-substituted-N,N'-diacyl hydrazine having the formula

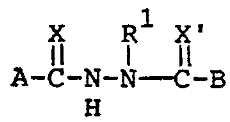

wherein

X and X′ are the same or different O, S or STR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitro; cyano; hydroxy; ($C_1$-$C_6$)alkyl, halo-($C_1$-$C_6$)alkyl; cyano-($C_1$-$C_6$)alkyl; ($C_1$-$C_6$)alkoxy; halo-($C_1$-$C_6$)alkoxy; ($C_1$-$C_6$)alkoxy-($C_1$-$C_6$)alkyl having independently the stated number of carbon atoms in each alkyl group; ($C_1$-$C_6$)alkoxy-($C_1$-$C_6$)alkoxy having independently the stated number of carbon atoms in each alkyl group; -$OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; carboxy; -$ZCO_2R'$ group; -COR group; halo-($C_1$-$C_6$)alkyl-carbonyl; cyano-($C_1$-$C_6$)alkyl-carbonyl; ($C_1$-$C_6$)-nitro-($C_1$-$C_6$)alkyl-carbonyl; ($C_1$-$C_6$)alkoxy-carbonyl; halo-($C_1$-$C_6$)alkoxy-carbonyl; -OCOR group; $NH_2$; NHZ; NZZ′; amino substituted with hydroxy, ($C_1$-$C_4$)alkoxy or ($C_1$-$C_4$)alkylthio groups; -CONRR′ group; -OCONRR′ group; -NRCOR′ group; -$NRCO_2R'$ group; -OCONRCOR′ group; sulfhydryl; ($C_1$-$C_6$)alkylthio; halo-($C_1$-$C_6$)alkylthio; -NRCSR′ group; -SCOR group; unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; thio-($C_1$-$C_6$)alkoxy; carboxy; ($C_1$-$C_6$)alkoxy-carbonyl; -$ZCO_2R'$ group; -CONRR′ group; amino; -NRCOR′ group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl, $NH_2$, NHZ or NZZ′;

where R and R ′ are hydrogen or ($C_1$-$C_6$)alkyl; Z and Z′ are ($C_1$-$C_6$)alkyl; "amino" means NRR′; and one of A or B is an unsubstituted or substituted six-membered heterocycle as defined above; provided that B is not 4-alkyl-substituted phenyl or 3-alkoxy-substituted phenyl when A is 2-pyridyl; or an agronomically acceptable salt thereof; together with agronomically acceptable diluent or carrier.

2. A composition according to claim 1 wherein

X and X′ are O or S;

$R^1$ is branched ($C_3$-$C_8$)alkyl; and

A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$) alkoxy-($C_1$-$C_4$)alkyl having independently the stated number of carbon atoms in each alkyl group; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or

unsubstituted or substituted six-membered heterocycle having one or two nitrogen atoms and 4 to 5 nuclear carbon atoms where the substituents can be one or two of the same or different halo; nitro; ($C_1$-$C_4$)alkyl; ($C_1$-$C_4$)alkoxy; thio-($C_1$-$C_4$)alkoxy; -NDD′; or unsubstituted or substituted phenyl having one or two of the same or different halo, nitro, ($C_1$-$C_4$)alkyl, halo-($C_1$-$C_4$)alkyl, ($C_1$-$C_4$)alkoxy, halo-($C_1$-$C_4$)alkoxy, carboxy or -NDD′;

where D and D' are hydrogen or $(C_1-C_4)$alkyl.

3. A composition according to claim 2 wherein

X and X' are O;

$R^1$ is branched $(C_4-C_7)$alkyl; and

A and B are phenyl or substituted phenyl where the substituents can be from one to three of the same or different halo, nitro, $(C_1-C_4)$alkyl, $(C_1-C_4)$alkoxy, or halo-$(C_1-C_4)$alkyl; or

unsubstituted or substituted six-membered heterocycle having one or two nitrogen atoms and four or five nuclear carbon atoms where the substituents can be one or two of the same or different halo, nitro, $(C_1-C_4)$alkyl, $(C_1-C_4)$alkoxy or thio-$(C_1-C_4)$alkoxy.

4. A composition according to claim 3 wherein

X and X' are O;

$R^1$ is t-butyl, neopentyl (2,2-dimethylpropyl) or 1,2,2-trimethylpropyl; and

A and B are phenyl or substituted phenyl where the substituents can be one or two of the same or different chloro, fluoro, bromo, iodo, nitro, methyl, ethyl or trifluoromethyl; or

unsubstituted or substituted pyridyl or 2,5-pyrazinyl where the substituent can be halo, nitro, $(C_1-C_4)$alkyl, $(C_1-C_4)$alkoxy or thio-$(C_1-C_4)$alkoxy.

5. A composition according to claim 4 wherein

X and X' are O;

$R^1$ is t-butyl; and

A and B are phenyl or substituted phenyl where the substituents can be one or two of the same or different chloro, fluoro, bromo, iodo, nitro, methyl, ethyl or trifluoromethyl; or pyridyl or 2,5-pyrazinyl, or pyridyl substituted with halo, $(C_1-C_3)$alkyl or thiomethoxy.

6. A composition according to claim 5 wherein

X and X' are O;

$R^1$ is t-butyl; and

A is 2-pyridyl and B is phenyl, 3-methylphenyl, 4-fluorophenyl or 2-chloro-4-fluorophenyl; or

A is 5-bromo-3-pyridyl and B is 4-chlorophenyl; or

A is 2-chloro-3-pyridyl and B is 3-methylphenyl; or

A is 2,3-dimethylphenyl and B is 2-chloro-3-pyridyl.

7. A composition according to any preceding claim containing said compound in an amount of 0.0001 to 99%.

8. A composition according to claim 7 containing said compound in an amount of 0.001 to about 90% by weight of the composition.

9. A composition according to claim 8 containing said compound in an amount of from 0.01 to 75% by weight of the composition.

10. An insecticidal composition according to claim 7 or 8 (a) additionally containing a dispersing agent, said composition being in the form of a wettable powder, or (b) containing a liquid agronomically acceptable carrier and a dispersing agent, said composition being in the form of a flowable, or (c) in the form of a dust, or (d) additionally containing a binding agent, said composition being in the form of a granule, or (e) additionally containing an attractant agent, said composition being in the form of a bait or (f) additionally containing an emulsifying agent, said composition being in the form of an emulsifiable concentrate.

11. A method of controlling insects which comprises contacting said insects with an effective amount of the insecticidally active compound as defined in any of claims 1 to 6 optionally in a composition according to any preceding.

12. A method according to claim 11 which comprises applying the compound or composition to growing plants or to an area where plants are to be grown at a rate of from 10 grams to 10 kilograms of the active compound per hectare, preferably from 100 grams to 5 kilograms per hectare.

13. A method according to claim 12 wherein said insects are from the order Lepidoptera.

**Claims for the following Contracting State: ES**

1. The use of an insecticidally active compound which is an N'-substituted-N,N'-diacyl hydrazine having the formula

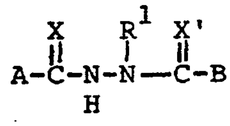

wherein

X and X′ are the same or different O, S or NR;

$R^1$ is unsubstituted ($C_3$-$C_{10}$) branched alkyl or a ($C_1$-$C_4$) straight chain alkyl substituted with one or two of the same or different ($C_3$-$C_6$)cycloalkyl; and

A and B are unsubstituted or substituted phenyl where the substituents can be from one to five of the same or different halo; nitro; cyano; hydroxy; ($C_1$-$C_6$)alkyl, halo-($C_1$-$C_6$)alkyl; cyano-($C_1$-$C_6$)alkyl; ($C_1$-$C_6$)alkoxy; halo-($C_1$-$C_6$)alkoxy; ($C_1$-$C_6$)alkoxy-($C_1$-$C_6$)alkyl having independently the stated number of carbon atoms in each alkyl group; ($C_1$-$C_6$)alkoxy-($C_1$-$C_6$)alkoxy having independently the stated number of carbon atoms in each alkyl group; -$OCO_2R$ group; ($C_2$-$C_6$)alkenyl optionally substituted with halo, cyano, ($C_1$-$C_4$)alkyl, ($C_1$-$C_4$)alkoxy, halo-($C_1$-$C_4$)alkoxy or ($C_1$-$C_4$)alkylthio; ($C_2$-$C_6$)alkenyl-carbonyl; ($C_2$-$C_6$)alkadienyl; ($C_2$-$C_6$)alkynyl optionally substituted with halo, cyano, nitro, hydroxy, ($C_1$-$C_4$)alkyl, ($C_1$-$C_4$)alkoxy, halo-($C_1$-$C_4$)alkoxy or ($C_1$-$C_4$)alkylthio; carboxy; -$ZCO_2R'$ group; -COR group; halo-($C_1$-$C_6$)alkyl-carbonyl; cyano-($C_1$-$C_6$)alkyl-carbonyl; ($C_1$-$C_6$)-nitro($C_1$-$C_6$)alkyl-carbonyl; ($C_1$-$C_6$)alkoxy-carbonyl; halo-($C_1$-$C_6$)alkoxy-carbonyl; -OCOR group; $NH_2$; NHZ; NZZ′; amino substituted with hydroxy, ($C_1$-$C_4$)alkoxy or ($C_1$-$C_4$)alkylthio groups; -CONRR′ group; -OCONRR′ group; -NRCOR′ group; -$NRCO_2R'$ group; -OCONRCOR′ group; sulfhydryl; ($C_1$-$C_6$)alkylthio; halo-($C_1$-$C_6$)alkylthio; -NRCSR′ group; -SCOR group; unsubstituted or substituted phenyl having one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or amino; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; benzoyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenoxycarbonyl where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxy-carbonyl, ($C_1$-$C_4$)alkanoyloxy, $NH_2$, NHZ or NZZ′; or when two adjacent positions on the phenyl ring are substituted with alkoxy groups, these groups may be joined to form, together with the carbon atoms to which they are attached, a 5 or 6 membered dioxolano or dioxano heterocyclic ring; or

unsubstituted or substituted six-membered heterocycle having one, two, three or four nitrogen atoms and two to five nuclear carbon atoms where the substituents can be from one to three of the same or different halo; nitro; hydroxy; ($C_1$-$C_6$)alkyl; ($C_1$-$C_6$)alkoxy; thio-($C_1$-$C_6$)alkoxy; carboxy; ($C_1$-$C_6$)alkoxy-carbonyl; -$ZCO_2R'$ group; -CONRR′ group; amino; -NRCOR′ group; ($C_1$-$C_6$)alkylthio; or unsubstituted or substituted phenyl having one to three of the same or different halo, nitro, ($C_1$-$C_6$)alkyl, halo-($C_1$-$C_6$)alkyl, ($C_1$-$C_6$)alkoxy, halo-($C_1$-$C_6$)alkoxy, carboxy, ($C_1$-$C_4$)alkoxy-carbonyl, $NH_2$, NHZ or NZZ′;

where R and R ′ are hydrogen or ($C_1$-$C_6$)alkyl; Z and Z′ are ($C_1$-$C_6$)alkyl; "amino" means NRR′; and one of A or B is an unsubstituted or substituted six-membered heterocycle as defined above; provided that B is not 4-alkyl-substituted phenyl or 3-alkoxy-substituted phenyl when A is 2-pyridyl; or an agronomically acceptable salt thereof, together with agronomically acceptable diluent or carrier to make an insecticidal composition.

2. The use according to claim 1 of the compound or salt wherein

X and X′ are O or S;

$R^1$ is branched ($C_3$-$C_8$)alkyl; and

A and B are unsubstituted or substituted phenyl having one to three of the same or different halo; nitro; cyano; ($C_1$-$C_4$)alkyl; halo-($C_1$-$C_4$)alkyl; cyano-($C_1$-$C_4$)alkyl; ($C_1$-$C_4$)alkoxy; ($C_1$-$C_4$) alkoxy-($C_1$-$C_4$)alkyl having independently the stated number of carbon atoms in each alkyl group; -COD; ($C_1$-$C_4$)alkoxy-carbonyl; ($C_1$-$C_4$)alkanoyloxy; unsubstituted or substituted phenyl having one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)alkoxy-carbonyl, ($C_1$-$C_4$)alkanoyloxy or -NDD′; or

unsubstituted or substituted six-membered heterocycle having one or two nitrogen atoms and 4 to 5 nuclear carbon atoms where the substituents can be one or two of the same or different halo; nitro; ($C_1$-$C_4$)alkyl; ($C_1$-$C_4$)alkoxy; thio-($C_1$-$C_4$)alkoxy; -NDD′; or unsubstituted or substituted phenyl having one or two of the same or

different halo, nitro, $(C_1-C_4)$alkyl, halo$(C_1-C_4)$alkyl, $(C_1-C_4)$alkoxy, halo-$(C_1-C_4)$alkoxy, carboxy or -NDD′; where D and D′ are hydrogen or $(C_1-C_4)$alkyl.

3. The use according to claim 2 of the compound or salt wherein
X and X′ are O;
$R^1$ is branched $(C_4-C_7)$alkyl; and
A and B are phenyl or substituted phenyl where the substituents can be from one to three of the same or different halo, nitro, $(C_1-C_4)$alkyl, $(C_1-C_4)$alkoxy, or halo-$(C_1-C_4)$alkyl; or
unsubstituted or substituted six-membered heterocycle having one or two nitrogen atoms and four or five nuclear carbon atoms where the substituents can be one or two of the same or different halo, nitro, $(C_1-C_4)$alkyl, $(C_1-C_4)$alkoxy or thio-$(C_1-C_4)$alkoxy.

4. The use according to claim 3 of the compound or salt wherein
X and X′ are O;
$R^1$ is t-butyl, neopentyl (2,2-dimethylpropyl) or 1,2,2-trimethylpropyl; and
A and B are phenyl or substituted phenyl where the substituents can be one or two of the same or different chloro, fluoro, bromo, iodo, nitro, methyl, ethyl or trifluoromethyl; or
unsubstituted or substituted pyridyl or 2,5-pyrazinyl where the substituent can be halo, nitro, $(C_1-C_4)$alkyl, $(C_1-C_4)$alkoxy or thio-$(C_1-C_4)$alkoxy.

5. The use according to claim 4 of the compound or salt wherein
X and X′ are O;
$R^1$ is t-butyl; and
A and B are phenyl or substituted phenyl where the substituents can be one or two of the same or different chloro, fluoro, bromo, iodo, nitro, methyl, ethyl or trifluoromethyl; or pyridyl or 2,5-pyrazinyl, or pyridyl substituted with halo, $(C_1-C_3)$alkyl or thiomethoxy.

6. The use according to claim 5 of the compound or salt wherein
X and X′ are O;
$R^1$ is t-butyl; and
A is 2-pyridyl and B is phenyl, 3-methylphenyl, 4-fluorophenyl or 2-chloro-4-fluorophenyl; or
A is 5-bromo-3-pyridyl and B is 4-chlorophenyl; or
A is 2-chloro-3-pyridyl and B is 3-methylphenyl; or
A is 2,3-dimethylphenyl and B is 2-chloro-3-pyridyl.

7. The use according to any preceding claim of the compound or salt in an amount of 0.0001 to 99%, preferably 0.001 to about 90% by weight of the composition.

8. The use according to claim 7 of the compound or salt in an amount of from 0.01 to 75% by weight of the composition.

9. The use according to any preceding claim of the compound or salt to make an insecticidal composition (a) additionally containing a dispersing agent, said composition being in the form of a wettable powder, or (b) containing a liquid agronomically acceptable carrier and a dispersing agent, said composition being in the form of a flowable, or (c) in the form of a dust, or (d) additionally containing a binding agent, said composition being in the form of a granule, or (e) additionally containing an attractant agent, said composition being in the form of a bait or (f) additionally containing an emulsifying agent, said composition being in the form of an emulsifiable concentrate.

10. The use of a compound or salt as defined in any of claims 1 to 6, optionally in a composition also containing agronomically acceptable diluent or carrier, for controlling insects, by contacting said insects with an insecticidally effective amount of said compound or salt.

11. A mechanical process for improving the commercial value and/or profitability of vendible crops from plants whose growth is affected or likely to be affected by insects comprising (1) charging to a container, fumigation device or mechanical dissemination device an insecticidal compound or salt as defined in any of claims 1 to 6, optionally in a mixture with agronomically acceptable diluent or carrier, (2) using the container, fumigator or mechanical dissemination device to apply the insecticidal compound or salt, in the form of granules, dust, smoke, vapour or surfactant-containing liquid preparation to growing plants or to a growth medium where the plants are growing or are to be grown, or to the insects themselves, (3) controlling the dose of the active ingredient during this application step so that the rate of application of active insecticidal compound is sufficient to combat the insects but is insufficient to cause an unacceptably adverse effect on the crop plants growing or to be grown in the treated area.

12. The use or process according to claim 10 or 11 which comprises applying the compound or composition to growing plants or to an area where plants are to be grown at a rate of from 10 grams to 10 kilograms of the active compound per hectare, preferably from 100 grams to 5 kilograms per hectare.

13. The use or process according to claim 12 wherein said insects are from the order Lepidoptera.

## Patentansprüche

### Patentansprüche für folgende Vertragsstaaten: BE, CH, DE, FR, GB, GR, IT, LI, LU, NL, SE

1. Insektizid wirksame, N'-substituierte N,N'-Diacylhydrazinverbindung mit der Formel:

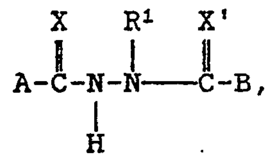

wobei

X und X' gleich oder unterschiedlich die Bedeutung O, S oder NR haben;

$R^1$ ein unsubstituiertes verzweigtes ($C_3$-$C_{10}$)-Alkyl oder ein geradkettiges ($C_1$-$C_4$)-Alkyl bedeutet, welches gleich oder unterschiedlich substituiert ist mit einem oder zwei ($C_3$-$C_6$)-Cycloalkyl; und

A und B bedeuten: unsubstituiertes oder substituiertes Phenyl, wobei die Substituenten gleich oder unterschiedlich sein können von eins bis fünf aus Halo; Nitro; Cyano; Hydroxy; ($C_1$-$C_6$)-Alkyl; Halo-($C_1$-$C_6$)-alkyl; Cyano-($C_1$-$C_6$)-alkyl; ($C_1$-$C_6$)-Alkoxy; Halo-($C_1$-$C_6$)-alkoxy; ($C_1$-$C_6$)-Alkoxy-($C_1$-$C_6$)-alkyl mit unabhängig der angegebenen Anzahl an Kohlenstoffatomen in jeder Alkylgruppe; ($C_1$-$C_6$)-Alkoxy-($C_1$-$C^6$)-alkoxy mit unabhängig der angegebenen Anzahl an Kohlenstoffatomen in jeder Alkylgruppe; -$OCO_2R$-Gruppe; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio; ($C_2$-$C_6$)-Alkenylcarbonyl; ($C_2$-$C_6$)-Alkadienyl; ($C_2$-$C_6$)-Alkynyl, wahlweise substituiert mit Halo, Cyano, Nitro, Hydroxy, ($C_1$-$C_4$)-Alkyl, ($C_1$-$C_4$)-Alkoxy, Halo-($C_1$-$C_4$)-alkoxy oder ($C_1$-$C_4$)-Alkylthio; Carboxy; -$ZCO_2R'$-Gruppe; -COR-Gruppe; Halo-($C_1$-$C_6$)-alkylcarbonyl; Cyano-($C_1$-$C_6$)-alkylcarbonyl; ($C_1$-$C_6$)-Nitro-($C_1$-$C_6$)-alkylcarbonyl; ($C_1$-$C_6$)-Alkoxycarbonyl; Halo-($C_1$-$C_6$)-alkoxycarbonyl; -OCOR-Gruppe; $NH_2$; NHZ; NZZ'; Amino, substituiert mit Hydroxy-, ($C_1$-$C_4$)-Alkoxy- oder ($C_1$-$C_4$)-Alkylthiogruppen; -CONRR'-Gruppe; -OCONRR'-Gruppe; -NRCOR'-Gruppe; -$NRCO_2R'$-Gruppe; -OCONRCOR'-Gruppe, Sulfhydryl; ($C_1$-$C_6$)-Alkylthio; Halo-($C_1$-$C_6$)-alkylthio; -NRCSR'-Gruppe; -SCOR-Gruppe; unsubstituiertes oder substituiertes Phenyl mit gleich oder unterschiedlich eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder Amino; Phenoxy, wobei oder Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; Benzoyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; Phenoxycarbonyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; Phenylthio, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; oder, wenn zwei benachbarte Positionen am Phenylring mit Alkoxygruppen substituiert sind, diese Gruppen untereinander verbunden sein können, um zusammen mit den Kohlenstoffatomen, an denen sie hängen, einen fünf- oder sechsgliedrigen heterocyclischen Dioxolan- oder Dioxanring zu bilden; oder

einen unsubstituierten oder substituierten sechsgliedrigen Heterocyclus mit eins, zwei, drei oder vier Stickstoffatomen und zwei bis fünf Kohlenstoffatomen im Kern, wobei die Substituenten gleich oder unterschiedlich sein können von eins bis drei aus Halo; Nitro; Hydroxy, ($C_1$-$C_6$)-Alkyl; ($C_1$-$C_6$)-Alkoxy; Thio-($C_1$-$C_6$)-alkoxy; Carboxy, ($C_1$-$C_6$)-Alkoxycarbonyl; -$ZCO_2R'$-Gruppen; -CONRR'Gruppen; Amino; -NRCOR'-Gruppen; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl, $NH_2$, NHZ oder NZZ';

wobei R und R' die Bedeutung Wasserstoff oder ($C_1$-$C_6$)-Alkyl haben; Z und Z' die Bedeutung ($C_1$-$C_6$)-Alkyl haben; "Amino" die Bedeutung NRR' hat; und eines von A oder B einen unsubstituierten oder substituierten sechsgliedrigen, wie vorstehend definierten Heterocyclus bedeutet; mit der Maßgabe, daß B nicht die Bedeutung 4-alkyl substituiertes Phenyl oder 3-alkoxy substituiertes Phenyl hat, wenn A die Bedeutung 2-Pyridyl hat; oder ein landwirtschaftlich vertretbares Salz davon.

2. Verbindung nach Anspruch 1, wobei

X und X' die Bedeutung O oder S haben;

$R^1$ ein verzweigtes ($C_3$-$C_8$)-Alkyl bedeutet; und

A und B bedeuten: unsubstituiertes oder substituiertes Phenyl mit gleich oder unterschiedlich eins bis drei aus Halo; Nitro; Cyano; ($C_1$-$C_4$)-Alkyl; Halo-($C_1$-$C_4$)-alkyl; Cyano-($C_1$-$C_4$)-alkyl; ($C_1$-$C_4$)-Alkoxy; ($C_1$-$C_4$)-Alkoxy-($C_1$-$C_4$)-alkyl mit unabhängig der angegebenen Anzahl an Kohlenstoffatomen in jeder Alkylgruppe; -COD; ($C_1$-$C_4$)-Alkoxycarbonyl; ($C_1$-$C_4$)-Alkanoyloxy; unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder -NDD'; oder Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder -NDD'; oder

einen unsubstituierten oder substituierten sechsgliedrigen Heterocyclus mit einem-oder zwei Stickstoffatomen und vier bis fünf Kohlenstoffatomen im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy,; -NDD'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NDD';

wobei D und D' die Bedeutung Wasserstoff oder ($C_1$-$C_4$)-Alkyl haben.

3. Verbindung nach Anspruch 2, wobei

X und X' die Bedeutung O haben;

$R^1$ ein verzweigtes ($C_4$-$C_7$)-Alkyl bedeutet; und

A und B bedeuten: Phenyl oder substituiertes Phenyl, wobei die Substituenten gleich oder unterschiedlich von eins bis drei aus Halo, Nitro, ($C_1$-$C_4$)-Alkyl, ($C_1$-$C_4$)-Alkoxy, oder Halo-($C_1$-$C_4$)-alkyl sein können; oder

einen unsubstituierten oder substituierten sechsgliedrigen Heterocyclus mit eins oder zwei Stickstoffatomen und vier oder fünf Kohleristoffatomen im Kern, wobei die Substituenten gleich oder unterschiedlich eins oder zwei aus Halo, Nitro, ($C_1$-$C_4$)-Alkyl, ($C_1$-$C_4$)-Alkoxy oder Thio-($C_1$-$C_4$)-alkoxy sein können.

4. Verbindung nach Anspruch 3, wobei

X und X' die Bedeutung O haben;

$R^1$ die Bedeutung t-Butyl, Neopentyl (2,2-Dimethylpropyl) oder 1,2,2-Trimethylpropyl hat; und

A und B bedeuten: Phenyl oder substituiertes Phenyl, wobei die Substituenten gleich oder unterschiedlich eins oder zwei aus Chlor, Fluor, Brom lod, Nitro, Methyl, Ethyl oder Trifluormethyl sein können; oder

unsubstituiertes oder substituiertes Pyridyl oder 2,5-Pyrazinyl, wobei die Substituenten Halo, Nitro, ($C_1$-$C_4$)-Alkyl, ($C_1$-$C_4$)-Alkoxy oder Thio-($C_1$-$C_4$)-alkoxy sein können.

5. Verbindung nach Anspruch 4, wobei

X und X' die Bedeutung O haben;

$R^1$ die Bedeutung t-Butyl hat; und

A und B bedeuten: Phenyl oder substituiertes Phenyl, wobei die Substituenten gleich oder unterschiedlich eins oder zwei aus Chlor, Fluor, Brom, lod, Nitro, Methyl, Ethyl oder Trifluormethyl; oder Pyridyl oder 2,5-Pyrazinyl oder Pyridyl, substituiert mit Halo, ($C_1$-$C_3$)-Alkyl oder Thiomethoxy sein können.

6. Verbindung nach Anspruch 5, wobei

X und X' die Bedeutung O haben;

$R^1$ die Bedeutung t-Butyl hat; und

A die Bedeutung 2-Pyridyl hat und B die Bedeutung Phenyl, 3-Methylphenyl, 4-Fluorphenyl oder 2-Chlor-4-fluorphenyl hat; oder

A die Bedeutung 5-Brom-3-pyridyl hat und B die Bedeutung 4-Chlorphenyl hat; oder

A die Bedeutung 2-Chlor-3-pyridyl hat und B die Bedeutung 3-Methylphenyl hat; oder

A die Bedeutung 2,3-Dimethylphenyl hat und B die Bedeutung 2-Chlor-3-pyridyl hat.

7. Insektizide Zusammensetzung, enthaltend eine insektizid wirksame Verbindung nach einem der vorhergehenden Ansprüche und landwirtschaftlich vertretbaren Verdünnungs-oder Trägerstoff.

8. Zusammensetzung nach Anspruch 7, enthaltend die Verbindung in einer Menge von 0,0001 bis 99 Gew.-% der Zusammensetzung, bevorzugt 0,001 bis etwa 90 %.

9. Zusammensetzung nach Anspruch 8, enthaltend die Verbindung in einer Menge von 0,01 bis 75 Gew.-% der Zusammensetzung.

10. Insektizide Zusammensetzung nach Anspruch 7 oder 8, welche (a) zusätzlich ein Dispersionsmittel enthält, wobei die Zusammensetzung in Form eines benetzbaren Pulvers vorliegt, oder (b) einen flüssigen, landwirtschaftlich vertretbaren Trägerstoff und ein Dispersionsmittel enthält, wobei die Zusammensetzung in fließbarer Form vorliegt, oder (c) in Form eines Staubs vorliegt, oder (d) zusätzlich ein bindemittel enthält, wobei die Zusammensetzung in Form von Granulat vorliegt, oder (e) zusätzlich einen Lockstoff enthält, wobei die Zusammensetzung in Form eines Köders vorliegt, oder (f) zusätzlich ein Emulgiermittel enthält, wobei die Zusammensetzung in Form eines emulgierbaren Konzentrats vorliegt.

11. Verfahren zum Kontrollieren von Insekten, bei dem man die Insekten mit einer wirksamen Menge der insektiziden Verbindung nach einem der Ansprüche 1 bis 6 in Kontakt bringt, wahlweise in einer Zusammensetzung nach einem der Ansprüche 7 bis 10.

12. Verfahren nach Anspruch 11, wobei man die Verbindung oder Zusammensetzung wachsenden Pflanzen oder einem Gebiet, in dem die Pflanzen zu ziehen sind, in einer Menge von 10 g bis 10 kg der Verbindung pro Hektar zusetzt, bevorzugt von 100 g bis 5 kg pro Hektar.

13. Verfahren nach Anspruch 12, wobei die Insekten von der Ordnung Lepidoptera sind.

**Patentansprüche für folgenden Vertragsstaat: AT**

1. Insektizid wirkende Zusammensetzung, enthaltend eine insektizid wirksame, N′-substituierte N,N′-Diacylhydrazinverbindung mit der Formel:

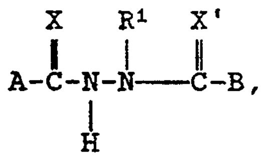

wobei

X und X′ gleich oder unterschiedlich die Bedeutung O, S oder NR haben;

$R^1$ ein unsubstituiertes verzweigtes ($C_3$-$C_{10}$)-Alkyl oder ein geradkettiges ($C_1$-$C_4$)-Alkyl bedeutet, welches gleich oder unterschiedlich substituiert ist mit einem oder zwei ($C_3$-$C_6$)-Cycloalkyl; und

A und B bedeuten: unsubstituiertes oder substituiertes Phenyl, wobei die Substituenten gleich oder unterschiedlich sein können von eins bis fünf aus Halo; Nitro; Cyano; Hydroxy; ($C_1$-$C_6$)-Alkyl; Halo-($C_1$-$C_6$)-alkyl; Cyano-($C_1$-$C_6$)-alkyl; ($C_1$-$C_6$)-Alkoxy; Halo-($C_1$-$C_6$)-alkoxy; ($C_1$-$C_6$)-Alkoxy-($C_1$-$C_6$)-alkyl mit unabhängig der angegebenen Anzahl an Kohlenstoffatomen in jeder Alkylgruppe; ($C_1$-$C_6$)-Alkoxy-($C_1$-$C^6$)-alkoxy mit unabhängig der angegebenen Anzahl an Kohlenstoffatomen in jeder Alkylgruppe; -$OCO_2R$-Gruppe; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio; ($C_2$-$C_6$)-Alkenylcarbonyl; ($C_2$-$C_6$)-Alkadienyl; ($C_2$-$C_8$)-Alkynyl, wahlweise substituiert mit Halo, Cyano, Nitro, Hydroxy, ($C_1$-$C_4$)-Alkyl, ($C_1$-$C_4$)-Alkoxy, Halo-($C_1$-$C_4$)-alkoxy oder ($C_1$-$C_4$)-Alkylthio; Carboxy; -$ZCO_2R'$-Gruppe; -COR-Gruppe; Halo-($C_1$-$C_6$)-alkylcarbonyl; Cyano-($C_1$-$C_6$)-alkylcarbonyl; ($C_1$-$C_6$)-Nitro-($C_1$-$C_6$)-alkylcarbonyl; ($C_1$-$C_6$)-Alkoxycarbonyl; Halo-($C_1$-$C_6$)-alkoxycarbonyl; -OCOR-Gruppe; $NH_2$; NHZ; NZZ′; Amino, substituiert mit Hydroxy-, ($C_1$-$C_4$)-Alkoxy- oder ($C_1$-$C_4$)-Alkylthiogruppen; -CONRR′-Gruppe; -OCONRR′-Gruppe; -NRCOR′-Gruppe; -$NRCO_2R'$-Gruppe; -OCONRCOR′-Gruppe, Sulfhydryl; ($C_1$-$C_6$)-Alkylthio; Halo-($C_1$-$C_6$)-alkylthio; -NRCSR′-Gruppe; -SCOR-Gruppe; unsubstituiertes oder substituiertes Phenyl mit gleich oder unterschiedlich eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder Amino; Phenoxy, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′; Benzoyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′; Phenoxycarbonyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′; Phenylthio, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ′; oder, wenn zwei benachbarte Positionen am Phenylring mit Alkoxygruppen substituiert sind, diese Gruppen miteinander verbunden sein können, um zusammen mit den Kohlenstoffatomen, an denen sie hängen, einen fünf-oder sechsgliedrigen heterocyclischen Dioxolan- oder Dioxanring zu bilden; oder

einen unsubstituierten oder substituierten sechsgliedrigen Heterocyclus mit eins, zwei, drei oder vier Stickstoffatomen und zwei bis fünf Kohlenstoffatomen im Kern, wobei die Substituenten gleich oder unterschiedlich sein können von eins bis drei aus Halo; Nitro; Hydroxy, ($C_1$-$C_6$)-Alkyl; ($C_1$-$C_6$)-Alkoxy; Thio-($C_1$-$C_6$)-alkoxy; Carboxy, ($C_1$-$C_6$)-Alkoxycarbonyl; -$ZCO_2R'$-Gruppen; -CONRR′-Gruppen; Amino; -NRCOR′-Gruppen; ($C_1$-$C_6$)-Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl, $NH_2$, NHZ oder NZZ′;

wobei R und R′ die Bedeutung Wasserstoff oder ($C_1$-$C_6$)-Alkyl haben; Z und 2′ die Bedeutung ($C_1$-$C_6$)-Alkyl haben; "Amino" die Bedeutung NRR′ hat; und eines von A oder B einen unsubstituierten oder substituierten sechsgliedrigen, wie vorstehend definierten Heterocyclus bedeutet; mit der Maßgabe, daß B nicht die Bedeutung 4-alkyl substituiertes Phenyl oder 3-alkoxy substituiertes Phenyl hat, wenn A die Bedeutung 2-Pyridyl hat; oder ein landwirtschaftlich vertretbares Salz davon; zusammen mit landwirtschaftlich vertretbarem Verdünnungs- oder Trägerstoff.

2. Zusammensetzung nach Anspruch 1, wobei

X und X′ die Bedeutung O oder S haben;

$R^1$ ein verzweigtes ($C_3$-$C_8$)-Alkyl bedeutet; und

A und B bedeuten: unsubstituiertes oder substituiertes Phenyl mit gleich oder unterschiedlich eins bis drei aus Halo; Nitro; Cyano; ($C_1$-$C_4$)-Alkyl; Halo-($C_1$-$C_4$)-alkyl; Cyano-($C_1$-$C_4$)-alkyl; ($C_1$-$C_4$)-Alkoxy; ($C_1$-$C_4$)-Alkoxy-($C_1$-$C_4$)-alkyl mit unabhängig der angegebenen Anzahl an Kohlenstoffatomen in jeder Alkylgruppe; -COD; ($C_1$-$C_4$)-Alkoxycarbonyl; ($C_1$-$C_4$)-Alkanoyloxy; unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder -NDD′; oder Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)Alkoxy, Carboxy, ($C_1$-$C_4$)-Alkoxycarbonyl, ($C_1$-$C_4$)-Alkanoyloxy oder -NDD′; oder

einen unsubstituierten oder substituierten sechsgliedrigen Heterocyclus mit einem oder zwei Stickstoffatomen und vier bis fünf Kohlenstoffatomen im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy,; -NDD′; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NDD′; wobei D und D′ die Bedeutung Wasserstoff oder ($C_1$-$C_4$)-Alkyl haben.

3. Zusammensetzung nach Anspruch 2, wobei

X und X′ die Bedeutung O haben;

$R^1$ ein verzweigtes ($C_4$-$C_7$)-Alkyl bedeutet; und

A und B bedeuten: Phenyl oder substituiertes Phenyl, wobei die Substituenten gleich oder unterschiedlich von eins bis drei aus Halo, Nitro, ($C_1$-$C_4$)-Alkyl, ($C_1$-$C_4$)-Alkoxy, oder Halo-($C_1$-$C_4$)-alkyl sein können; oder

einen unsubstituierten oder substituierten sechsgliedrigen Heterocyclus mit eins oder zwei Stickstoffatomen und vier oder fünf Kohlenstoffatomen im Kern, wobei die Substituenten gleich oder unterschiedlich eins oder zwei aus Halo, Nitro, ($C_1$-$C_4$)-Alkyl, ($C_1$-$C_4$)-Alkoxy oder Thio-($C_1$-$C_4$)-alkoxy sein können.

4. Zusammensetzung nach Anspruch 3, wobei

X und X′ die Bedeutung O haben;

$R^1$ die Bedeutung t-Butyl, Neopentyl (2,2-Dimethylpropyl) oder 1,2,2-Trimethylpropyl hat; und

A und B bedeuten: Phenyl oder substituiertes Phenyl, wobei die Substituenten gleich oder unterschiedlich eins oder zwei aus Chlor, Fluor, Brom Iod, Nitro, Methyl, Ethyl oder Trifluormethyl sein können; oder

unsubstituiertes oder substituiertes Pyridyl oder 2,5-Pyradinyl, wobei die Substituenten Halo, Nitro, ($C_1$-$C_4$)-Alkyl, ($C_1$-$C_4$)-Alkoxy oder Thio-($C_1$-$C_4$)-alkoxy sein können.

5. Zusammensetzung nach Anspruch 4, wobei

X und X′ die Bedeutung O haben;

$R^1$ die Bedeutung t-Butyl hat; und

A und B bedeuten: Phenyl oder substituiertes Phenyl, wobei die Substituenten gleich oder unterschiedlich eins oder zwei aus Chlor, Fluor, Brom, Iod, Nitro, Methyl, Ethyl oder Trifluormethyl; oder Pyridyl oder 2,5-Pyrazinyl oder Pyridyl, substituiert mit Halo, ($C_1$-$C_3$)-Alkyl oder Thiomethoxy sein können.

6. Zusammensetzung nach Anspruch 5, wobei

X und X′ die Bedeutung O haben;

$R^1$ die Bedeutung t-Butyl hat; und

A die Bedeutung 2-Pyridyl hat und B die Bedeutung Phenyl, 3-Methylphenyl, 4-Fluorphenyl oder 2-Chlor-4-fluorphenyl hat; oder

A die Bedeutung 5-Brom-3-pyridyl hat und B die Bedeutung 4-Chlorphenyl hat; oder

A die Bedeutung 2-Chlor-3-pyridyl hat und B die Bedeutung 3-Methylphenyl hat; , oder

A die Bedeutung 2,3-Dimethylphenyl hat und B die Bedeutung 2-Chlor-3-pyridyl hat.

7. Zusammensetzung nach einem der vorhergehenden Ansprüche enthaltend die Verbindung in einer Menge von 0,0001 bis 99 %.

8. Zusammensetzung nach Anspruch 7, enthaltend die Verbindung in einer Menge von 0,001 bis etwa 90 Gew.-% der Zusammensetzung.

9. Zusammensetzung nach Anspruch 8, enthaltend die Verbindung in einer Menge von 0,01 bis 75 Gew.-% der Zusammensetzung.

10. Insektizide Zusammensetzung nach Anspruch 7 oder 8, welche (a) zusätzlich ein Dispersionsmittel enthält, wobei die Zusammensetzung in Form eines benetzbaren Pulvers vorliegt, oder (b) einen flüssigen, landwirtschaftlich vertretbaren Trägerstoff und ein Dispersionsmittel enthält, wobei die Zusammensetzung in

fließbarer Form vorliegt, oder (c) in Form eines Staubs vorliegt, oder (d) zusätzlich ein Bindemittel enthält, wobei die Zusammensetzung in Form von Granulat vorliegt, oder (e) zusätzlich einen Lockstoff enthält, wobei die Zusammensetzung in Form eines Köders vorliegt, oder (f) zusätzlich ein Emulgiermittel enthält, wobei die Zusammensetzung in Form eines emulgierbaren Konzentrats vorliegt.

11. Verfahren zum Kontrollieren von Insekten, bei dem man die Insekten mit einer wirksamen Menge der insektiziden Verbindung wie in einem der Ansprüche 1 bis 6 definiert in Kontakt bringt, wahlweise in einer Zusammensetzung nach einem der Ansprüche 7 bis 10.

12. Verfahren nach Anspruch 11, wobei man die Verbindung oder Zusammensetzung wachsenden Pflanzen oder einem Gebiet, in dem die Pflanzen zu ziehen sind, in einer Menge von 10 g bis 10 kg der Verbindung pro Hektar zusetzt, bevorzugt von 100 g bis 5 kg pro Hektar.

13. Verfahren nach Anspruch 12, wobei die Insekten von der Ordnung Lepidoptera sind.

**Patentansprüche für folgenden Vertragsstaat: ES**

1. Verwendung einer insektizid wirksamen, N'-substituierten N,N'-Diacylhydrazinverbindung mit der Formel:

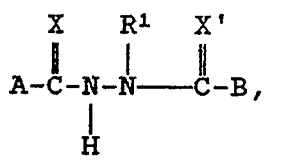

wobei

X und X' gleich oder unterschiedlich die Bedeutung O, S oder NR haben;

$R^1$ ein unsubstituiertes verzweigtes ($C_3$-$C_{10}$)-Alkyl oder ein geradkettiges ($C_1$-$C_4$)-Alkyl bedeutet, welches gleich oder unterschiedlich substituiert ist mit einem oder zwei ($C_3$-$C_6$)-Cycloalkyl; und

A und B bedeuten: unsubstituiertes oder substituiertes Phenyl, wobei die Substituenten gleich oder unterschiedlich sein können von eins bis fünf aus Halo; Nitro; Cyano; Hydroxy; ($C_1$-$C_6$)-Alkyl; Halo-($C_1$-$C_6$)-alkyl; Cyano-($C_1$-$C_6$)-alkyl; ($C_1$-$C_6$)-Alkoxy; Halo-($C_1$-$C_6$)-alkoxy; ($C_1$-$C_6$)-Alkoxy-($C_1$-$C_6$)-alkyl mit unabhängig der angegebenen Anzahl an Kohlenstoffatomen in jeder Alkylgruppe; ($C_1$-$C_6$)-Alkoxy-($C_1$-$C_6$)-alkoxy mit unabhängig der angegebenen Anzahl an Kohlenstoffatomen in jeder Alkylgruppe; -$OCO_2$R-Gruppe; ($C_2$-$C_6$)-Alkenyl, wahlweise substituiert mit Halo, Cyano, ($C_1$-$C_4$)-Alkyl, ($C_1$-$C_4$)-Alkoxy, Halo-($C_1$-$C_4$)-alkoxy oder ($C_1$-$C_4$)-Alkylthio; ($C_2$-$C_6$)-Alkenylcarbonyl; ($C_2$-$C_6$)-Alkadienyl; ($C_2$-$C_6$)-Alkynyl, wahlweise substituiert mit Halo, Cyano, Nitro, Hydroxy, ($C_1$-$C_4$)-Alkyl, ($C_1$-$C_4$)-Alkoxy, Halo-($C_1$-$C_4$)-alkoxy oder ($C_1$-$C_4$)-Alkylthio; Carboxy; -$ZCO_2$R'-Gruppe; -COR-Gruppe; Halo-($C_1$-$C_6$)-alkylcarbonyl; Cyano-($C_1$-$C_6$)-alkylcarbonyl; ($C_1$-$C_6$)-Nitro-($C_1$-$C_6$)-alkylcarbonyl; ($C_1$-$C_6$)-Alkoxycarbonyl; Halo-($C_1$-$C_6$)-alkoxycarbonyl; -OCOR-Gruppe; $NH_2$; NHZ; NZZ'; Amino, substituiert mit Hydroxy-, ($C_1$-$C_4$)-Alkoxy- oder ($C_1$-$C_4$)-Alkylthiogruppen; -CONRR'-Gruppe; -OCONRR'-Gruppe; -NRCOR'-Gruppe; -$NRCO_2$R'-Gruppe; -OCONRCOR'-Gruppe, Sulfhydryl; ($C_1$-$C_6$)-Alkylthio; Halo-($C_1$-$C_6$)-alkylthio; -NRCSR'-Gruppe; -SCOR-Gruppe; unsubstituiertes oder substituiertes Phenyl mit gleich oder unterschiedlich eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder Amino; Phenoxy, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$2, NHZ oder NZZ'; Benzoyl, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; Phenoxycarbonyl, wobei der Phenylring unsubstituiert ist oder gleich oder unterschiedlich substituiert ist mit eins bis drei aus kalo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; Phenylthio, wobei der Phenylring unsubstituiert ist oder gleich oder unterschiedlich substituiert ist mit eins bis drei aus Halo, Cyano, Nitro, Hydroxy, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy, $NH_2$, NHZ oder NZZ'; oder, wenn zwei benachbarte Positionen am Phenylring mit Alkoxygruppen substituiert sind, diese Gruppen untereinander verbunden sein können, um zusammen mit den Kohlenstoffatomen, an denen sie hängen, einen fünf- oder sechsgliedrigen heterocyclischen Dioxolan- oder Dioxanring zu bilden; oder

einen unsubstituierten oder substituierten sechsgliedrigen Heterocyclus mit eins, zwei, drei oder vier Stickstoffatomen und zwei bis fünf Kohlenstoffatomen im Kern, wobei die Substituenten gleich oder unterschiedlich sein können von eins bis drei aus Halo; Nitro; Hydroxy, ($C_1$-$C_6$)-Alkyl; ($C_1$-$C_6$)-Alkoxy; Thio-($C_1$-$C_6$)-alkoxy; Carboxy, ($C_1$-$C_6$)-Alkoxycarbonyl; -$ZCO_2$R'-Gruppen; -CONRR'-Gruppen; Amino; -NRCOR'-Gruppen; ($C_1$-$C_6$)-

Alkylthio; oder unsubstituiertes oder substituiertes Phenyl mit gleich oder unterschiedlich eins bis drei aus Halo, Nitro, ($C_1$-$C_6$)-Alkyl, Halo-($C_1$-$C_6$)-alkyl, ($C_1$-$C_6$)-Alkoxy, Halo-($C_1$-$C_6$)-alkoxy, Carboxy, ($C_1$-$C_4$)-Alkoxycarbonyl, $NH_2$, NHZ oder NZZ';

wobei R und R' die Bedeutung Wasserstoff oder ($C_1$-$C_6$)-Alkyl haben; Z und Z' die Bedeutung ($C_1$-$C_6$)-Alkyl haben; "Amino" die Bedeutung NRR' hat; und eines von A oder B einen unsubstituierten oder substituierten sechsgliedrigen, wie vorstehend definierten Heterocyclus bedeutet; mit der Maßgabe, daß B nicht die Bedeutung 4-alkyl substituiertes Phenyl oder 3-alkoxy substituiertes Phenyl hat, wenn A die Bedeutung 2-Pyridyl hat; oder eines landwirtschaftlich vertretbaren Salzes davon zusammen mit landwirtschaftlich vertretbarem Verdünnung- oder Trägerstoff zum Herstellen einer insektiziden Zusammensetzung.

2. Verwendung nach Anspruch 1 der Verbindung oder des Salzes, wobei

X und X' die Bedeutung O oder S haben;

$R^1$ ein verzweigtes ($C_3$-$C_8$)-Alkyl bedeutet; und

A und B bedeuten: unsubstituiertes oder substituiertes Phenyl mit gleich oder unterschiedlich eins bis drei aus Halo; Nitro; Cyano; ($C_1$-$C_4$)-Alkyl; Halo-($C_1$-$C_4$)-alkyl; Cyano-($C_1$-$C_4$)-alkyl; ($C_1$-$C_4$)-Alkoxy; ($C_1$-$C_4$)-Alkoxy-($C_1$-$C_4$)-alkyl mit unabhängig der angegebenen Anzahl an Kohlenstoffatomen in jeder Alkylgruppe; -COD; ($C_1$-$C_4$)-Alkoxycarbonyl; ($C_1$-$C_4$)-Alkanoyloxy; unsubstituiertes oder substituiertes Phenyl mit gleich oder unterschiedlich eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder -NDD'; oder Phenoxy, wobei der Phenylring unsubstituiert ist oder gleich oder unterschiedlich substituiert ist mit eins oder zwei aus Halo, Nitro, ($C_1$-$C_4$)-Alkyl, ($C_1$-$C_4$)-Alkoxy, Carboxy, ($C_1$-$C_4$)-Alkoxycarbonyl, ($C_1$-$C_4$)-Alkanoyloxy oder -NDD'; oder

einen unsubstituierten oder substituierten sechsgliedrigen Heterocyclus mit einem oder zwei Stickstoffatomen und vier bis fünf Kohlenstoffatomen im Kern, wobei die Substituenten gleich oder unterschiedlich sein können eins oder zwei aus Halo; Nitro; ($C_1$-$C_4$)-Alkyl; ($C_1$-$C_4$)-Alkoxy; Thio-($C_1$-$C_4$)-alkoxy,; -NDD'; oder unsubstituiertes oder substituiertes Phenyl mit gleich oder unterschiedlich eins oder zwei aus Halo, Nitro, ($C_1$-$C_4$)-Alkyl, Halo-($C_1$-$C_4$)-alkyl, ($C_1$-$C_4$)-Alkoxy, Halo-($C_1$-$C_4$)-alkoxy, Carboxy oder -NDD'; wobei D und D' die Bedeutung Wasserstoff oder ($C_1$-$C_4$)-Alkyl haben.

3. Verwendung nach Anspruch 2 der Verbindung oder des Salzes, wobei

X und X' die Bedeutung O haben;

$R^1$ ein verzweigtes ($C_4$-$C_7$)-Alkyl bedeutet; und

A und B bedeuten: Phenyl oder substituiertes Phenyl, wobei die Substituenten gleich oder unterschiedlich von eins bis drei aus Halo, Nitro, ($C_1$-$C_4$)-Alkyl, ($C_1$-$C_4$)-Alkoxy, oder Halo-($C_1$-$C_4$)-alkyl sein können; oder

einen unsubstituierten oder substituierten sechsgliedrigen Heterocyclus mit eins oder zwei Stickstoffatomen und vier oder fünf Kohlenstoffatomen im Kern, wobei die Substituenten gleich oder unterschiedlich eins oder zwei aus Halo, Nitro, ($C_1$-$C_4$)-Alkyl, ($C_1$-$C_4$)-Alkoxy oder Thio-($C_1$-$C_4$)-alkoxy sein können.

4. Verwendung nach Anspruch 3 der Verbindung oder des Salzes, wobei

X und X' die Bedeutung O haben;

$R^1$ die Bedeutung t-Butyl, Neopentyl (2,2-Dimethylpropyl) oder 1,2,2-Trimethylpropyl hat; und

A und B bedeuten: Phenyl oder substituiertes Phenyl, wobei die Substituenten gleich oder unterschiedlich eins oder zwei aus Chlor, Fluor, Brom Iod, Nitro, Methyl, Ethyl oder Trifluormethyl sein können; oder

unsubstituiertes oder substituiertes Pyridyl oder 2,5-Pyrazinyl, wobei die Substituenten Halo, Nitro, ($C_1$-$C_4$)-Alkyl, ($C_1$-$C_4$)-Alkoxy oder Thio-($C_1$-$C_4$)-alkoxy sein können.

5. Verwendung nach Anspruch 4 der Verbindung oder des Salzes, wobei

X und X' die Bedeutung O haben;

$R^1$ die Bedeutung t-Butyl hat; und

A und B bedeuten: Phenyl oder substituiertes Phenyl, wobei die Substituenten gleich oder unterschiedlich eins oder zwei aus Chlor, Fluor, Brom, Iod, Nitro, Methyl, Ethyl oder Trifluormethyl; oder Pyridyl oder 2,5-Pyrazinyl oder Pyridyl, substituiert mit Halo, ($C_1$-$C_3$)-Alkyl oder Thiomethoxy sein können.

6. Verwendung nach Anspruch 5 der Verbindung oder des Salzes, wobei

X und X' die Bedeutung O haben;

$R^1$ die Bedeutung t-Butyl hat; und

A die Bedeutung 2-Pyridyl hat und B die Bedeutung Phenyl, 3-Methylphenyl, 4-Fluorophenyl oder 2-Chlor-4-fluorphenyl hat; oder

A die Bedeutung 5-Brom-3-pyridyl hat und B die Bedeutung 4-Chlorphenyl hat; oder

A die Bedeutung 2-Chlor-3-pyridyl hat und B die Bedeutung 3-Methylphenyl hat; oder

A die Bedeutung 2,3-Dimethylphenyl hat und B die Bedeutung 2-Chlor-3-pyridyl hat.

7. Verwendung nach einem der vorgehenden Ansprüche der Verbindung oder des Salzes in einer Menge von 0,0001 bis 99 Gew.-% der Zusammensetzung, bevorzugt 0,001 bis etwa 90 %.

8. Verwendung nach Anspruch 7 der Verbindung oder des Salzes in einer Menge von 0,01 bis 75 Gew.-%

der Zusammensetzung.

9. Verwendung nach einem der vorhergehenden Ansprüche der Verbindung oder des Salzes zum Herstellen einer insektiziden Zusammensetzung, welche (a) zusätzlich ein Dispersionsmittel enthält, wobei die Zusammensetzung in Form eines benetzbaren Pulvers vorliegt, oder (b) einen flüssigen, landwirtschaftlich vertretbaren Trägerstoff und ein Dispersionsmittel enthält, wobei die Zusammensetzung in fließbarer Form vorliegt, oder (c) in Form eines Staubs vorliegt, oder (d) zusätzlich ein Bindemittel enthält, wobei die Zusammensetzung in Form von Granulat vorliegt, oder (e) zusätzlich einen Lockstoff enthält, wobei die Zusammensetzung in Form eines Köders vorliegt, oder (f) zusätzlich ein Emulgiermittel enthält, wobei die Zusammensetzung in Form eines emulgierbaren Konzentrats vorliegt.

10. Verwendung einer Verbindung oder eines Salzes wie in einem der Ansprüche 1 bis 6 definiert, wahlweise in einer Zusammensetzung, die auch landwirtschaftlich vertretbaren Verdünnung- oder Trägerstoff enthält, zum Kontrollieren von Insekten, wobei man die Insekten mit einer insektizid wirksamen Menge der Verbindung oder des Salzes im Kontakt bringt.

11. Mechanisches Verfahren zur Verbesserung des Handelswertes und/oder der Rentabilität verkaufbarer Ernten aus Pflanzen, deren Wachstum von Insekten beeinflußt wird oder möglicherweise beeinflußt wird, wobei man (1) einem Behälter, einer Vernebelungsvorrichtung oder einem mechanischem Ausbreiter eine insektizide Verbindung oder ein Salz wie in einem der Ansprüche 1 bis 6 definiert zuführt, wahlweise in einer Mischung mit landwirtschaftlich vertretbarem Verdünnungs- oder Trägerstoff, (2) den Behälter, Vernebeler oder mechanischen Ausbreiter dazu verwendet, die insektizide Verbindung oder das Salz in Form von Granulat, Staub, Rauch, Dampf oder tensidhaltiger Flüssigkeitszubereitung wachsenden Pflanzen oder einem Wachstumsmedium zuzuführen, wo die pflanzen wachsen oder zu ziehen sind, oder den Insekten selbst, (3) die Dosis des aktiven Bestandteiles während des Applizierungsschrittes so steuert, daß die Applizierungsrate der wirksamen Insektizidverbindung zum Bekämpfen der Insekten ausreicht, jedoch nicht ausreicht zur Erzeugung einer nicht hinnehmbaren nachteiligen Auswirkung auf die in dem behandelten Gebiet wachsenden oder zu ziehenden Erntepflanzen.

12. Verwendung oder Verfahren nach einem der Ansprüche 10 oder 11, wobei man die Verbindung oder Zusammensetzung wachsenden Pflanzen oder einem Gebiet, in dem die Pflanzen zu ziehen sind, in einer Menge von 10 g bis 10 kg der Verbindung pro Hektar zusetzt, bevorzugt von 100 g bis 5 kg pro Hektar.

13. Verwendung oder Verfahren nach Anspruch 12, wobei die Insekten von der Ordnung Lepidoptera sind.

**Revendications**

**Revendications pour les Etats contractants suivants: BE, CH, DE, FR, GB, GR, IT, LI, LU, NL, SE**

1. Un composé insecticidement actif qui est une N,N'-diacyl hydrazine, N'-substituée, ayant la formule

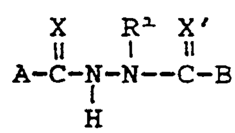

dans laquelle

X et X', les mêmes ou différents, sont O, S ou NR ;

$R^1$ est un alcoyl ramifié en $C_3$-$C_{10}$ non substitué ou un alcoyl à chaîne droite en $C_1$-$C_4$ substitué par un ou deux groupes, les mêmes ou différents, cycloalcoyl en $C_3$-$C_6$ ; et

A et B sont un groupe phényl non substitue ou substitué dans lequel les substituants peuvent être de 1 à 5, les mêmes ou différents, halo ; nitro; cyano ; hydroxy ; alcoyl en $C_1$-$C_6$, halo-alcoyl en $C_1$ à $C_6$ ; cyano-alcoyl en $C_1$-$C_6$ ; aalcoxy en $C_1$ à $C_6$ ; haloalcoxy en $C_1$ à $C_6$ ; alcoxy (en $C_1$-$C_6$)-alcoyl en $C_1$ à $C_6$ ayant indépendamment le nombre indiqué d'atomes de carbone dans chaque groupe alcoyl ; alcoxy en $C_1$ à $C_6$-alcoxy en $C_1$ à $C_6$ ayant indépendamment le nombre indiqué d'atomes de carbone dans chaque groupe alcoyl ; groupe -$OCO_2R$ ; alcényl en $C_2$-$C_6$ éventuellement substitué par halo , cyano, alcoyl en $C_1$-$C_4$, alcoxy en $C_1$-$C_4$, halo-alcoxy en $C_1$-$C_4$ ou alcoylthio en $C_1$ à $C_4$, alcényl (en $C_1$ à $C_6$)-carbonyl ; alcadiényl en $C_2$-$C_6$ ; alcoynyl en $C_2$ à $C_6$ éventuellement substitué par halo, cyano, nitro, hydroxy, alcoyl en $C_1$-$C_4$, alcoxy en $C_1$ à $C_4$, haloalcoxy en $C_1$ à $C_4$ ou alcoylthio en $C_1$ à $C_4$ ; carboxy ; groupe -$ZCO_2R'$ ; groupe -COR ; halo-alcoyl (en $C_1$ à $C_4$)-carbonyl ; cyano-alcoyl (en $C_1$ à $C_6$)-carbonyl;($C_1$-$C_6$)-nitro-alcoyl (en $C_1$ à $C_6$)-carbonyl; alcoxy-carbonyl ; halo-

alcoxy (en $C_1$ à $C_6$)-carbonyl; -groupe OCOR ; $NH_2$; NHZ ; NZZ' ; groupes amino substitués par hydroxy, alcoxy en $C_1$-$C_4$ ou alcoylthio en $C_1$-$C_4$ ; groupe -CONRR' ; groupe -OCONRR' ; groupe -NRCOR' ; groupe -$NRCO_2R'$; groupe -OCONRCOR' ; sulfhydryl ; alcoylthio en $C_1$-$C_6$ ; halo-alcoylthio en $C_1$ à $C_6$ ; groupe -NRCSR' ; groupe -SCOR ; phényl non substitué ou substitué ayant un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoxy en $C_1$ à $C_4$ ou amino ; phénoxy dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; benzoyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénoxycarbonyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénylthio dans lequel le cycle phényl est non substitué ou substitue par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; ou, lorsque deux positions adjacentes du cycle phényl sont substituées par des groupes alcoxy, ces groupes peuvent être réunis pour former ensemble avec les atomes de carbone auxquels ils sont attaches, un hétérocycle dioxolano ou dioxano à cinq ou six éléments ; ou un hétérocycle à six éléments, non substitué ou substitué, ayant un, deux, trois ou quatre atomes d'azote et deux à cinq atomes de carbone nucléaire, dans lequel les substituants peuvent être de un à trois, les mêmes ou différents, halo ; nitro ; hydroxy ; alcoyl en $C_1$ à $C_8$, alcoxy en $C_1$ à $C_6$, thio-alcoxy en $C_1$ à $C_6$ ; carboxy ; alcoxy (en $C_1$ à $C_8$)-carbonyl ; groupe -$ZCO_2R'$ ; groupe -CONRR' ; amino ; groupe -NRCOR' ; alcoylthio en $C_1$ à $C_8$ ; ou phényl non substitué ou substitué ayant un à trois, les mêmes ou différents, halo, nitro, alcoxyl en $C_1$ à $C_8$, halo-alcoyl en $C_1$ à $C_8$, alcoxy en $C_1$ à $C_6$, halo-alcoxy en $C_1$ à $C_6$, carboxy, alcoxy en ($C_1$ à $C_4$)-carbonyl, $NH_2$, NHZ ou NZZ' ;

dans lesquels R et R' sont hydrogène ou alcoyl en $C_1$ à $C_8$ ; Z et Z' sont alcoyl en $C_1$ à $C_8$ ; "amino" désigne NRR' ; et l'un de A ou B est un hétérocycle, non substitué ou substitué, à six éléments comme défini ci-dessus ; à condition que B ne soit pas un phényl substitué par alcoyl en position 4 ou un phényl substitué par alcoxy en position 3 lorsque A est 2-pyridyl ; ou un sel agronomiquement acceptable de ceux-ci.

2. Un composé selon la revendication 1 dans lequel

X et X' sont O ou S ;

$R^1$ est un alcoyl ramifié en $C_3$-$C_8$ ; et

A et B sont phényl non substitué ou substitué ayant un à trois, les memes ou différents, halo ; nitro ; cyano ; alcoyl en $C_1$ à $C_4$ ; halo-alcoyl en $C_1$ à $C_4$ cyano-alcoyl en $C_1$ à $C_4$, alcoxy en $C_1$ à $C_4$, alcoxy (en $C_1$ à $C_4$)-alcoyl en $C_1$ à $C_4$ ayant indépendamment le nombre indiqué d'atomes de carbone dans chaque groupe alcoyl ; -COD ; alcoxy (en $C_1$ à $C_4$)-carbonyl; alcanoyloxy en $C_1$ à $C_4$ ; phényl non substitué ou substitué ayant un ou deux, les mêmes ou différents, halo, nitro, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$ ou -NDD' ; ou phénoxy dans lequel le cycle phényl est non substitué ou substitué par un ou deux, les mêmes ou différents, halo, nitro, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$ ou -NDD' ; ou

un hétérocycle à six éléments, non substitué ou substitué, ayant un ou deux atomes d'azote et quatre à cinq atomes de carbone nucléaire dans lesquels les substituants peuvent être un ou deux, les mêmes ou différents, halo ; nitro ; alcoyl en $C_1$ à $C_4$ ; alcoxy en $C_1$ à $C_4$, thioalcoxy en $C_1$ à $C_4$; -NDD' ; ou phényl non substitué ou substitué ayant un ou deux, les mêmes ou différents, halo, nitro, alcoyl en $C_1$ à $C_4$, halo-alcoyl en $C_1$ à $C_4$ ; alcoxy en $C_1$ à $C_4$, halo-alcoxy en $C_1$ à $C_4$, carboxy ou -NND' ;

dans lesquels D et D' sont hydrogène ou alcoyl en $C_1$ à $C_4$.

3. Un composé selon la revendication 2 dans lequel

X et X' sont O ;

$R^1$ est un alcoyl ramifié en $C_1$ à $C_7$ ; et

A et B sont phényl ou phényl substitué dans lesquels les substituants peuvent être de un à trois, les mêmes ou différents, halo, nitro, alcoyl en $C_1$ à $C_4$, alcoxy en $C_1$ à $C_4$ ou halo-alcoyl en $C_1$ à $C_4$ ; ou

un hétérocycle à six éléments, non substitué ou substitué, ayant un ou deux atomes d'azote et quatre ou cinq atomes de carbone nucléaire, dans lesquels les substituants peuvent être un ou deux, les mêmes ou différents, halo, nitro, alcoyl en $C_1$ à $C_4$, alcoxy en $C_1$ à $C_4$ ou thio-alcoxy en $C_1$ à $C_4$.

4. Un composé selon la revendication 3, dans lequel

X et X' sont O ;

$R^1$ est t-butyl, néopentyl (2,2-diméthylpropyl) ou 1,2,2-triméthylpropyl ; et

A et B sont phényl ou phényl substitué dans lesquels les substituants peuvent être un ou deux, les mêmes ou différents, chloro, fluoro, bromo, iodo, nitro, méthyl, éthyl ou trifluorométhyl ; ou pyridyl ou 2,5-pyrazinyl, non

substitués ou substitués dans lesquels le substituant peut être halo, nitro, alcoyl en $C_1$ à $C_4$, alcoxy en $C_1$ à $C_4$, ou thio-alcoxy en $C_1$ à $C_4$.

5. Un composé selon la revendication 4, dans lequel

X et X′ sont O ;

$R^1$ est t-butyl ; et

A et B sont phényl ou phényl substitué dans lesquels les substituants peuvent être un ou deux, les mêmes ou différents, chloro, fluro, bromo, iodo, nitro, méthyl, éthyl ou trifluorométhyl ; ou pyridyl ou 2,5-pyrazinyl, ou pyridyl substitué par halo, alcoyl en $C_1$ à $C_3$ ou thiométhoxy.

6. Un composé selon la revendication 5, dans lequel

X et X′ sont O ;

$R^1$ est t-butyl ; et

A est 2-pyridyl et B est phényl, 3-méthylphényl, 4-fluorophényl ou 2-chloro-4-fluorophényl ; ou

A est 5-bromo-3-pyridyl et B est 4-chlorophényl ; ou

A est 2-chloro-3-pyridyl et B est 3-méthylphenyl; ou

A est 2,3-diméthylphényl et B est 2-chloro-3-pyridyl.

7. Une composition insecticide comprenant un composé insecticidement actif selon l'une quelconque des revendications précédentes et un diluant ou véhicule agronomiquement acceptable.

8. Une composition selon la revendication 7 contenant ledit composé en une quantité de 0,0001 à 99 %, de préférence de 0,001 à environ 90 %, en poids de la composition.

9. Une composition selon la revendication 8 contenant ledit composé en une quantité de 0,01 à 75 % en poids de la composition.

10. Une composition insecticide selon la revendication 7 ou 8 (a) contenant additionnellement un agent dispersant, ladite composition étant sous la forme d'une poudre mouillable, ou (b) contenant un véhicule liquide agronomiquement acceptable et un agent dispersant, ladite composition étant sous la forme d'un produit coulant, ou (c) sous la forme d'une poussière, ou (d) contenant en outre un agent liant, ladite composition étant sous la forme d'un granulé ou (e) contenant en outre un agent attractif, ladite composition étant sous la forme d'un appât ou (f) contenant en outre un agent emulsifiant, ladite composition étant sous la forme d'un concentré émulsifiable.

11. Une méthode pour contrôler les insectes qui comprend la mise en contact desdits insectes avec une quantité efficace du composé insecticidement actif selon l'une quelconque des revendications 1 à 6, éventuellement dans une composition selon l'une quelconque des revendications 7 à 10.

12. Une méthode selon la revendication 11 qui comprend l'application du composé ou de la composition à des plantes en croissance ou dans une zone dans laquelle des plantes sont cultivées, en une quantité de 10 g à 10 kg du composé actif par hectare, de préférence de 100 g à 5 kg par hectare.

13. Une méthode selon la revendication 12 dans laquelle les insectes sont de l'ordre des lépidoptères.

**Revendications pour l'Etat Contractant suivant: AT**

1. Une composition insecticide comprenant un composé insecticidement actif qui est une N,N′-diacyl hydrazine, N'substituée, ayant la formule

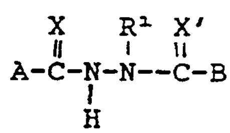

dans laquelle

X et X′, les mêmes ou différents, sont O, S ou NR ;

$R^1$ est un alcoyl ramifié en $C_3$-$C_{10}$ non substitué ou un alcoyl à chaîne droite en $C_1$-$C_4$ substitué par un ou deux groupes, les mêmes ou différents, cycloalcoyl en $C_3$-$C_6$ ; et

A et B sont un groupe phényl non substitué ou substitué dans lequel les substituants peuvent être de 1 à 5, les mêmes ou différents, halo ; nitro; cyano ; hydroxy ; alcoyl en $C_1$-$C_6$, halo-alcoyl en $C_1$ à $C_6$ ; cyano-alcoyl en $C_1$-$C_6$ ; aalcoxy en $C_1$ à $C_6$ ; haloalcoxy en $C_1$ à $C_6$ ; alcoxy (en $C_1$-$C_6$)-alcoyl en $C_1$ à $C_6$ ayant indépendamment le nombre indiqué d'atomes de carbone dans chaque groupe alcoyl ; alcoxy en $C_1$ à $C_6$-alcoxy en $C_1$ à $C_6$ ayant indépendamment le nombre indiqué d'atomes de carbone dans chaque groupe alcoyl ; groupe -$OCO_2R$ ; alcényl en $C_2$-$C_6$ éventuellement substitué par halo , cyano, alcoyl en $C_1$-$C_4$, alcoxy en $C_1$-$C_4$, haloalcoxy en $C_1$-$C_4$ ou alcoylthio en $C_1$ à $C_4$, alcényl (en $C_1$ à $C_6$)-carbonyl ; alcadiényl en $C_2$-$C_6$ ; alcoynyl en $C_2$

à $C_8$ éventuellement substitué par halo, cyano, nitro, hydroxy, alcoyl en $C_1$-$C_4$, alcoxy en $C_1$ à $C_4$, haloalcoxy en $C_1$ à $C_4$ ou alcoylthio en $C_1$ à $C_4$ ; carboxy ; groupe -$ZCO_2R'$ ; groupe -COR ; halo-alcoyl (en $C_1$ à $C_4$)-carbonyl ; cyano-alcoyl (en $C_1$ à $C_6$)-carbonyl;($C_1$-$C_6$)-nitro-alcoyl (en $C_1$ à $C_6$)-carbonyl; alcoxy-carbonyl ; halo-alcoxy (en $C_1$ à $C_6$)-carbonyl; -groupe OCOR ; $NH_2$; NHZ ; NZZ' ; groupes amino substitués par hydroxy, alcoxy en $C_1$-$C_4$ ou alcoylthio en $C_1$-$C_4$ ; groupe -CONRR' ; groupe -OCONRR' ; groupe -NRCOR' ; groupe -$NRCO_2R'$; groupe -OCONRCOR' ; sulfhydryl ; alcoylthio en $C_1$-$C_6$ ; halo-alcoylthio en $C_1$ à $C_4$ ; groupe -NRCSR' ; groupe -SCOR ; phényl non substitué ou substitué ayant un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoxy en $C_1$ à $C_4$ ou amino ; phénoxy dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; benzoyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénoxycarbonyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénylthio dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; ou, lorsque deux positions adjacentes du cycle phényl sont substituées par des groupes alcoxy, ces groupes peuvent être réunis pour former ensemble avec les atomes de carbone auxquels ils sont attachés, un hétérocycle dioxolano ou dioxano à cinq ou six éléments ; ou un hétérocycle à six éléments, non substitué ou substitué, ayant un, deux, trois ou quatre atomes d'azote et deux à cinq atomes de carbone nucléaire, dans lequel les substituants peuvent être de un à trois, les mêmes ou différents, halo ; nitro ; hydroxy ; alcoyl en $C_1$ à $C_6$, alcoxy en $C_1$ à $C_6$, thio-alcoxy en $C_1$ à $C_6$ ; carboxy ; alcoxy (en $C_1$ à $C_6$)-carbonyl ; groupe - $ZCO_2R'$ ; groupe -CONRR' ; amino ; groupe -NRCOR' ; alcoylthio en $C_1$ à $C_6$ ; ou phényl non substitue ou substitué ayant un à trois, les mêmes ou différents, halo, nitro, alcoxyl en $C_1$ à $C_6$, halo-alcoyl en $C_1$ à $C_6$, alcoxy en $C_1$ à $C_6$, halo-alcoxy en $C_1$ à $C_6$, carboxy, alcoxy en ($C_1$ à $C_4$)-carbonyl, $NH_2$, NHZ ou NZZ' ;

dans lesquels R et R' sont hydrogène ou alcoyl en $C_1$ à $C_6$ ; Z et Z' sont alcoyl en $C_1$ à $C_6$ ; "amino" désigne NRR' ; et l'un de A ou B est un hétérocycle, non substitué ou substitué, à six éléments comme défini ci-dessus ; à condition que B ne soit pas un phényl substitué par alcoyl en position 4 ou un phényl substitué par alcoxy en position 3 lorsque A est 2-pyridyl ; ou un sel agronomiquement acceptable de ceux-ci ; ensemble avec un diluant ou véhicule agronomiquement acceptable.

2. Une composition selon la revendication 1 dans laquelle

X et X' sont O ou S ;

$R^1$ est un alcoyl ramifié en $C_3$-$C_8$ ; et

A et B sont phényl non substitue ou substitué ayant un à trois,les mêmes ou différents, halo ; nitro ; cyano ; alcoyl en $C_1$ à $C_4$ ; halo-alcoyl en $C_1$ à $C_4$ cyano-alcoyl en $C_1$ à $C_4$, alcoxy en $C_1$ à $C_4$, alcoxy (en $C_1$ à $C_4$)-alcoyl en $C_1$ à $C_4$ ayant indépendamment le nombre indiqué d'atomes de carbone dans chaque groupe alcoyl ; -COD ; alcoxy (en $C_1$ à $C_4$)-carbonyl; alcanoyloxy en $C_1$ à $C_4$ ; phényl non substitué ou substitué ayant un ou deux, les mêmes ou différents, halo, nitro, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$ ou -NDD' ; ou phénoxy dans lequel le cycle phényl est non substitué ou substitué par un ou deux, les mêmes ou différents, halo, nitro, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$ ou -NDD' ; ou

un hétérocycle à six éléments, non substitué ou substitué, ayant un ou deux atomes d'azote et quatre à cinq atomes de carbone nucléaire dans lesquels les substituants peuvent être un ou deux, les mêmes ou différents, halo ; nitro ; alcoyl en $C_1$ à $C_4$ ; alcoxy en $C_1$ à $C_4$, thioalcoxy en $C_1$ à $C_4$; -NDD' ; ou phényl non substitué ou substitué ayant un ou deux, les mêmes ou différents, halo, nitro, alcoyl en $C_1$ à $C_4$, halo-alcoyl en $C_1$ à $C_4$ ; alcoxy en $C_1$ à $C_4$, halo-alcoxy en $C_1$ à $C_4$, carboxy ou -NND' ;

dans lesquels D et D' sont hydrogène ou alcoyl en $C_1$ à $C_4$.

3. Une composition selon la revendication 2 dans laquelle

X et X' sont O ;

$R^1$ est un alcoyl ramifié en $C_1$ à $C_7$ ; et

A et B sont phényl ou phényl substitue dans lesquels les substituants peuvent être de un à trois, les mêmes ou différents, halo, nitro, alcoyl en $C_1$ à $C_4$, alcoxy en $C_1$ à $C_4$ ou halo-alcoyl en $C_1$ à $C_4$ ; ou

un hétérocycle à six éléments, non substitué ou substitué, ayant un ou deux atomes d'azote et quatre ou cinq atomes de carbone nucléaire, dans lesquels les substituants peuvent être un ou deux, les mêmes ou différents, halo, nitro, alcoyl en $C_1$ à $C_4$, alcoxy en $C_1$ à $C_4$ ou thio-alcoxy en $C_1$ à $C_4$.

4. Une composition selon la revendication 3, dans laquelle

X et X′ sont O ;

$R^1$ est t-butyl, néopentyl (2,2-diméthylpropyl) ou 1,2,2-triméthylpropyl ; et

A et B sont phényl ou phényl substitué dans lesquels les substituants peuvent être un ou deux, les mêmes ou différents, chloro, fluoro, bromo, iodo, nitro, méthyl, éthyl ou trifluorométhyl ; ou pyridyl ou 2,5-pyrazinyl, non substitués ou substitués dans lesquels le substituant peut être halo, nitro, alcoyl en $C_1$ à $C_4$, alcoxy en $C_1$ à $C_4$, ou thio-alcoxy en $C_1$ à $C_4$.

5. Une composition selon la revendication 4, dans laquelle

X et X′ sont O ;

$R^1$ est t-butyl ; et

A et B sont phényl ou phényl substitue dans lesquels les substituants peuvent être un ou deux, les mêmes ou différents, chloro, fluro, bromo, iodo, nitro, méthyl, éthyl ou trifluorométhyl ; ou pyridyl ou 2,5-pyrazinyl, ou pyridyl substitué par halo, alcoyl en $C_1$ à $C_3$ ou thiométhoxy.

6. Une composition selon la revendication 5, dans laquelle

X et X′ sont O ;

$R^1$ est t-butyl ; et

A est 2-pyridyl et B est phényl, 3-méthylphényl, 4-fluorophényl ou 2-chloro-4-fluorophényl ; ou

A est 5-bromo-3-pyridyl et B est 4-chlorophényl ; ou

A est 2-chloro-3-pyridyl et B est 3-méthylphényl; ou

A est 2,3-diméthylphényl et B est 2-chloro-3-pyridyl.

7. Une composition selon l'une quelconque des revendications précédentes contenant ledit composé en une quantité de 0,0001 à 99 %.

8. Une composition selon la revendication 7 contenant ledit composé en une quantité de 0,001 à environ 90 % en poids de la composition.

9. Une composition selon la revendication 8 contenant ledit composé en une quantité de 0,01 à 75 % en poids de la composition.

10. Une composition insecticide selon la revendication 7 ou 8 (a) contenant additionnellement un agent dispersant, ladite composition étant sous la forme d'une poudre mouillable, ou (b) contenant un véhicule liquide agronomiquement acceptable et un agent dispersant, ladite composition étant sous la forme d'un produit coulant, ou (c) sous la forme d'une poussière, ou (d) contenant en outre un agent liant, ladite composition étant sous la forme d'un granulé ou (e) contenant en outre un agent attractif, ladite composition étant sous la forme d'un appât ou (f) contenant en outre un agent émulsifiant, ladite composition étant sous la forme d'un concentré émulsifiable.

11. Une méthode pour contrôler les insectes qui comprend la mise en contact desdits insectes avec une quantité efficace du composé insecticidement actif tel que défini dans l'une quelconque des revendications 1 à 6, éventuellement dans une composition selon l'une quelconque des revendications précédentes.

12. Une méthode selon la revendication 11 qui comprend l'application du composé ou de la composition à des plantes en croissance ou dans une zone dans laquelle des plantes sont cultivées, en une quantité de 10 g à 10 kg du composé actif par hectare, de préférence de 100 g à 5 kg par hectare.

13. Une méthode selon la revendication 12 dans laquelle les insectes sont de l'ordre des lépidoptères.

**Revendications pour l'Etat Contractant suivant: ES**

1. L'utilisation d'un composé insecticidement actif qui est une N,N′-diacyl hydrazine, N'-substituée, ayant la formule

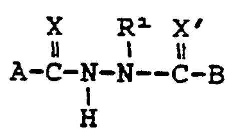

dans laquelle

X et X′, les mêmes ou différents, sont O, S ou NR ;

$R^1$ est un alcoyl ramifié en $C_3$-$C_{10}$ non substitué ou un alcoyl à chaîne droite en $C_1$-$C_4$ substitué par un ou deux groupes, les mêmes ou différents, cycloalcoyl en $C_3$-$C_6$ ; et

A et B sont un groupe phényl non substitué ou substitué dans lequel les substituants peuvent être de 1 à 5, les mêmes ou différents, halo ; nitro; cyano ; hydroxy ; alcoyl en $C_1$-$C_6$, halo-alcoyl en $C_1$ à $C_6$ ; cyano-alcoyl en $C_1$-$C_6$ ; aalcoxy en $C_1$ à $C_6$ ; haloalcoxy en $C_1$ à $C_6$ ; alcoxy (en $C_1$-$C_6$)-alcoyl en $C_1$ à $C_6$ ayant indépendamment le nombre indiqué d'atomes de carbone dans chaque groupe alcoyl ; alcoxy en $C_1$ à $C_6$-alcoxy en $C_1$ à

$C_6$ ayant indépendamment le nombre indiqué d'atomes de carbone dans chaque groupe alcoyl ; groupe -$OCO_2R$ ; alcényl en $C_2$-$C_6$ éventuellement substitué par halo , cyano, alcoyl en $C_1$-$C_4$, alcoxy en $C_1$-$C_4$, halo-alcoxy en $C_1$-$C_4$ ou alcoylthio en $C_1$ à $C_4$, alcényl (en $C_1$ à $C_6$)-carbonyl ; alcadiényl en $C_2$-$C_6$ ; alcoynyl en $C_2$ à $C_6$ éventuellement substitué par halo, cyano, nitro, hydroxy, alcoyl en $C_1$-$C_4$, alcoxy en $C_1$ à $C_4$, haloalcoxy en $C_1$ à $C_4$ ou alcoylthio en $C_1$ à $C_4$ ; carboxy ; groupe -$ZCO_2R'$ ; groupe -COR ; halo-alcoyl (en $C_1$ à $C_4$)-carbonyl ; cyano-alcoyl (en $C_1$ à $C_6$)-carbonyl;($C_1$-$C_6$)-nitro-alcoyl (en $C_1$ à $C_6$)-carbonyl; alcoxy-carbonyl ; halo-alcoxy (en $C_1$ à $C_6$)-carbonyl; -groupe OCOR ; $NH_2$; NHZ ; NZZ' ; groupes amino substitués par hydroxy, alcoxy en $C_1$-$C_4$ ou alcoylthio en $C_1$-$C_4$ ; groupe -CONRR' ; groupe -OCONRR' ; groupe -NRCOR' ; groupe -$NRCO_2R'$; groupe -OCONRCOR' ; sulfhydryl ; alcoylthio en $C_1$-$C_6$ ; halo-alcoylthio en $C_1$ à $C_6$ ; groupe -NRCSR' ; groupe -SCOR ; phényl non substitué ou substitué ayant un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoxy en $C_1$ à $C_4$ ou amino ; phénoxy dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; benzoyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénoxycarbonyl dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; phénylthio dans lequel le cycle phényl est non substitué ou substitué par un à trois, les mêmes ou différents, halo, cyano, nitro, hydroxy, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$, $NH_2$, NHZ ou NZZ' ; ou, lorsque deux positions adjacentes du cycle phényl sont substituées par des groupes alcoxy, ces groupes peuvent être réunis pour former ensemble avec les atomes de carbone auxquels ils sont attachés, un hétérocycle dioxolano ou dioxano à cinq ou six éléments ; ou un hétérocycle à six éléments, non substitué ou substitué, ayant un, deux, trois ou quatre atomes d'azote et deux à cinq atomes de carbone nucléaire, dans lequel les substituants peuvent être de un à trois, les mêmes ou différents, halo ; nitro ; hydroxy ; alcoyl en $C_1$ à $C_6$, alcoxy en $C_1$ à $C_6$, thio-alcoxy en $C_1$ à $C_6$ ; carboxy ; alcoxy (en $C_1$ à $C_6$)-carbonyl ; groupe -$ZCO_2R'$ ; groupe -CONRR' ; amino ; groupe -NRCOR' ; alcoylthio en $C_1$ à $C_6$ ; ou phényl non substitué ou substitué ayant un à trois, les mêmes ou différents, halo, nitro, alcoxyl en $C_1$ à $C_6$, halo-alcoyl en $C_1$ à $C_6$, alcoxy en $C_1$ à $C_6$, halo-alcoxy en $C_1$ à $C_6$, carboxy, alcoxy en ($C_1$ à $C_4$)-carbonyl, $NH_2$, NHZ ou NZZ' ;

dans lesquels R et R' sont hydrogène ou alcoyl en $C_1$ à $C_6$ ; Z et Z' sont alcoyl en $C_1$ à $C_6$ ; "amino" désigne NRR' ; et l'un de A ou B est un hétérocycle, non substitué ou substitué, à six éléments comme défini ci-dessus ; à condition que B ne soit pas un phényl substitué par alcoyl en position 4 ou un phényl substitué par alcoxy en position 3 lorsque A est 2-pyridyl ; ou un sel agronomiquement acceptable de celui-ci, ensemble avec un diluant ou véhicule agronomiquement acceptable pour réaliser une composition insecticide.

2. L'utilisation selon la revendication 1 du composé ou sel dans lequel

X et X' sont O ou S ;

$R^1$ est un alcoyl ramifié en $C_3$-$C_8$ ; et

A et B sont phényl non substitué ou substitué ayant un à trois,les mêmes ou différents, halo ; nitro ; cyano ; alcoyl en $C_1$ à $C_4$ ; halo-alcoyl en $C_1$ à $C_4$ cyano-alcoyl en $C_1$ à $C_4$, alcoxy en $C_1$ à $C_4$, alcoxy (en $C_1$ à $C_4$)-alcoyl en $C_1$ à $C_4$ ayant indépendamment le nombre indiqué d'atomes de carbone dans chaque groupe alcoyl ; -COD ; alcoxy (en $C_1$ à $C_4$)-carbonyl; alcanoyloxy en $C_1$ à $C_4$ : phényl non substitué ou substitué ayant un ou deux, les mêmes ou différents, halo, nitro, alcoyl en $C_1$ à $C_4$, alcoxy en $C_1$ à $C_4$, carboxy, alcoxy (en $C_1$ à $C_4$)-carbonyl, alcanoyloxy en $C_1$ à $C_4$ ou -NDD' ; ou phénoxy dans lequel le cycle phényl est non substitué ou substitué par un ou deux, les mêmes ou différents, halo, nitro, alcoyl en $C_1$ à $C_4$, alcoxy en C1 à C4, carboxy, alcoxy (en $C_1$ à $C_4$)-carbonyl, alcanoyloxy en $C_1$ à $C_4$ ou -NDD' ; ou

un hétérocycle à six éléments, non substitué ou substitué, ayant un ou deux atomes d'azote et quatre à cinq atomes de carbone nucléaire dans lesquels les substituants peuvent être un ou deux, les mêmes ou différents, halo ; nitro ; alcoyl en $C_1$ à $C_4$ ; alcoxy en $C_1$ à $C_4$, thioalcoxy en $C_1$ à $C_4$; -NDD' ; ou phényl non substitué ou substitué ayant un ou deux, les mêmes ou différents, halo, nitro, alcoyl en $C_1$ à $C_4$, halo-alcoyl en $C_1$ à $C_4$ ; alcoxy en $C_1$ à $C_4$, halo-alcoxy en $C_1$ à $C_4$, carboxy ou -NND' ;

dans lesquels D et D' sont hydrogène ou alcoyl en $C_1$ à $C_4$.

3. L'utilisation selon la revendication 2 du composé ou sel dans lequel

X et X' sont O :

$R^1$ est un alcoyl ramiéie en $C_1$ à $C_7$ ; et

A et B sont phényl ou phényl substitué dans lesquels les substituants peuvent être de un à trois, les mêmes ou différents, halo, nitro, alcoyl en $C_1$ à $C_4$, alcoxy en $C_1$ à $C_4$ ou halo-alcoyl en $C_1$ à $C_4$ ; ou

un hétérocycle à six éléments, non substitué ou substitué, ayant un ou deux atomes d'azote et quatre ou

cinq atomes de carbone nucléaire, dans lesquels les substituants peuvent être un ou deux, les memes ou différents, halo, nitro, alcoyl en $C_1$ à $C_4$, alcoxy en $C_1$ à $C_4$ ou thio-alcoxy en $C_1$ à $C_4$.

4. L'utilisation selon la revendication 3 du composé ou sel dans lequel

X et X' sont O ;

$R^1$ est t-butyl, ,néopentyl (2,2-diméthylpropyl) ou 1,2,2-triméthylpropyl ; et

A et B sont phényl ou phényl substitué dans lesquels les substituants peuvent être un ou deux, les mêmes ou différents, chloro, fluoro, bromo, iodo, nitro, méthyl, éthyl ou trifluorométhyl ; ou pyridyl ou 2,5-pyrazinyl, non substitués ou substitués dans lesquels le substituant peut être halo, nitro, alcoyl en $C_1$ à $C_4$, alcoxy en $C_1$ à $C_4$, ou thio-alcoxy en $C_1$ à $C_4$.

5. L'utilisation selon la revendication 4 du composé ou sel dans lequel

X et X' sont O ;

$R^1$ est t-butyl ; et

A et B sont phényl ou phényl substitué dans lesquels les substituants peuvent être un ou deux, les mêmes ou différents, chloro, fluro, bromo, iodo, nitro, méthyl, éthyl ou trifluorométhyl ; ou pyridyl ou 2,5-pyrazinyl, ou pyridyl substitué par halo, alcoyl en $C_1$ à $C_3$ ou thiométhoxy.

6. L'utilisation selon la revendication 5 du composé ou sel dans lequel

X et X' sont O ;

$R^1$ est t-butyl ; et

A est 2-pyridyl et B est phényl, 3-méthylphényl, 4-fluorophényl ou 2-chloro-4-fluorophényl ; ou

A est 5-bromo-3-pyridyl et B est 4-chlorophényl ; ou

A est 2-chloro-3-pyridyl et B est 3-méthylphényl; ou

A est 2,3-diméthylphenyl et B est 2-chloro-3-pyridyl.

7. L'utilisation selon l'une quelconque des revendications précédentes du composé ou sel en une quantité de 0,0001 à 99 %, de préférence de 0,001 à environ 90 %, en poids de la composition.

8. L'utilisation selon la revendication 7 du composé ou sel en une quantité de 0,01 à 75 % en poids de la composition.

9. L'utilisation selon l'une quelconque des revendications précédentes du composé ou sel pour réaliser une composition insecticide selon la revendication 7 ou 8 (a) contenant additionnellement un agent dispersant, ladite composition étant sous la forme d'une poudre mouillable, ou (b) contenant un vehicule liquide agronomiquement acceptable et un agent dispersant, ladite composition étant sous la forme d'un produit coulant, ou (c) sous la forme d'une poussière, ou (d) contenant en outre un agent liant, ladite composition étant sous la forme d'un granulé ou (e) contenant en outre un agent attractif, ladite composition étant sous la forme d'un appât ou (f) contenant en outre un agent émulsifiant, ladite composition étant sous la forme d'un concentré émulsifiable.

10. L'utilisation d'un composé ou sel tel que défini dans l'une quelconque des revendications 1 à 6, éventuellement dans une composition contenant également un diluant ou véhicule agronomiquement acceptable, pour contrôler les insectes, en mettant en contact lesdits insectes avec une quantité insecticidement efficace dudit composé ou sel.

11. Un procédé mécanique pour améliorer la valeur commerciale et/ou le profit tiré de récoltes vendables de plantes dont la croissance est affectée ou peut vraisemblablement être affectée par des insectes comprenant (1) le chargement dans un récipient, un dispositif de fumigation ou un dispositif de dissémination mécanique d'un composé ou sel insecticide tel que défini dans l'une quelconque des revendications 1 à 6, éventuellement en mélange avec un diluant ou véhicule agronomiquement acceptable, (2) l'utilisation du récipient, fumigateur ou dispositif de dissémination mécanique pour appliquer le composé ou sel insecticide, sous la forme de granules, poussière, fumée, vapeur ou préparation liquide contenant un agent tensio-actif à des plantes en croissance ou à un milieu de croissance dans lequel des plantes croissent ou vont croître, ou aux insectes eux-mêmes, (3) le contrôle de la dose de l'ingrédient actif pendant cette phase d'application pour que le taux d'application du composé insecticide actif soit suffisant pour combattre les insectes, mais soit insuffisant pour produire un effet adverse inacceptable sur les plantes de la récole croissant ou devant croître dans la zone traitée.

12. L'utilisation ou procédé selon la revendication 10 ou 11 qui comprend l'application du composé ou composition à des plantes en croissance ou dans une zone dans laquelle des plantes sont cultivées, en une quantité de 10 g à 10 kg du composé actif par hectare, de préférence de 100 g à 5 kg par hectare.

13. L'utilisation ou procédé selon la revendication 12 dans lequel les insectes sont de l'ordre des lépidoptères.